# EUROPÄISCHE PATENTSCHRIFT

(11) **EP 1 599 178 B1**
(45) Veröffentlichungstag und Bekanntmachung des Hinweises auf die Patenterteilung: **19.12.2007**
(21) Anmeldenummer: 04706649.3
(22) Anmeldetag: 30.01.2004
(51) Int. Cl.: A61K 8/49, A61Q 5/10

(54) **MITTEL UND VERFAHREN ZUM FÄRBEN VON KERATINFASERN**
MEANS AND METHOD FOR DYEING KERATIN FIBRES
AGENT ET PROCEDE DE COLORATION DE FIBRES KERATINIQUES

(30) Priorität: 05.03.2003 DE 10309521
(43) Veröffentlichungstag der Anmeldung: 30.11.2005
(73) Patentinhaber: Wella Aktiengesellschaft, 64274 Darmstadt (DE)
(72) Erfinder: PASQUIER, Cécile, CH-1723 Marly (CH); KIENER, Caroline, CH-1731 Ependes (CH); BRAUN, Hans-Jürgen, CH-3182 Ueberstorf (CH)
(86) Internationale Anmeldenummer: PCT/EP2004/000815
(87) Internationale Veröffentlichungsnummer: WO 2004/078151

(56) Entgegenhaltungen:
- WO-A-02/074267
- WO-A-02/074268
- DE-A- 1 922 400

## Beschreibung

Gegenstand der vorliegenden Anmeldung ist ein Mittel zur Färbung von Keratinfasern, wie zum Beispiel Seide, Wolle oder Haaren und insbesondere menschlichen Haaren, auf der Basis von Oxidationsfarbstoffvorstufen, welches mindestens ein heterozyklisches Hydrazon-Derivat der Formel (I) und mindestens ein Persulfatsalz als Oxidationsmittel enthält, ein Mehrkomponenten-Kit sowie ein Verfahren zum Färben von Keratinfasern unter Verwendung dieses Mittels.

Haarfärbemittel werden je nach zu färbender Ausgangshaarfarbe und gewünschtem Endresultat hauptsächlich in die Gruppe der Oxidationsfärbemittel oder der Tönungen unterteilt. Oxidationsfärbemittel eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von über 50 % oder zum "Hellerfärben" verwendeten Oxidationsfärbemittel in der Regel als sogenannte oxidative Farben bezeichnet werden. Direktziehende Farbstoffe sind hauptsächlich in nicht-oxidativen Färbemitteln (sogenannten Tönungsmitteln) enthalten. Einige direktziehende Farbstoffe, wie zum Beispiel Nitrofarbstoffe, können aufgrund ihrer geringen Größe in das Haar eindringen und es -zumindest in den äusseren Bereichen- direkt anfärben. Derartige Tönungen sind sehr haarschonend und überstehen in der Regel 6 bis 8 Haarwäschen. Direktziehende Farbstoffe werden ebenfalls oft in oxidativen Färbemitteln zur Erzeugung bestimmter Nuancen beziehungsweise zur Intensivierung der Farbe eingesetzt. Die bisher bekannten Färbesysteme können jedoch die an Färbemittel gestellten Anforderungen nicht in jeder Hinsicht, besonders im Hinblick auf Glanz und Intensität der Färbungen, erfüllen.

Die Verwendung von fünfringheterozyklischen Hydrazonderivaten einer bestimmten Struktur zur Färbung von Keratinfasern ist aus der WO-A 02074268 und DE-A 1922400 bekannt.

Überraschenderweise wurde nunmehr gefunden, daß bestimmte heterozyklische Hydrazonderivate mit üblichen Kupplersubstanzen, wie zum Beispiel aromatische Hydroxyl- und/ oder Aminogruppen enthaltenden Verbindungen, in Gegenwart von Persulfatsalzen intensive Färbungen im gelben bis blauen Farbbereich ermöglichen. Dieses neue Färbemittel gibt besonders brillante und intensive Färbungen, die eine sehr hohe Schweissbeständigkeit zeigen.

Gegenstand der vorliegenden Erfindung ist daher ein Mittel zur Färbung von Keratinfasern (A), wie zum Beispiel Wolle, Seide oder Haaren und insbesondere menschlichen Haaren, welches dadurch gekennzeichnet ist, dass es (a) mindestens ein Hydrazon-Derivat der Formel (I) oder dessen physiologisch verträgliches Salz, 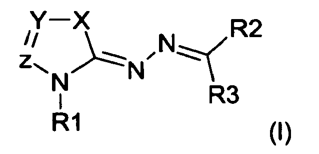 worin
**X** gleich Sauerstoff, Schwefel oder N-R4 ist;
**Y** gleich C-R5 oder Stickstoff ist;
**Z** gleich C-R6 oder Stickstoff ist;
mit der Bedingung, dass der heterozyklische Teil der Verbindung der Formel (I) maximal drei Heteroatome enthält;
**R1** und **R4** gleich oder verschieden sein können, und unabhängig voneinander eine gesättigte oder ungesättigte (C₁-C₁₂)-Alkylgruppe, eine mit einem Halogenatom (F, Cl, Br, J) substituierte (C₁-C₁₂)-Alkylgruppe, eine Hydroxy-(C₁-C₁₂)-alkylgruppe, eine Amino-(C₁-C₁₂)-alkylgruppe, eine Sulfonsäure-(C₁-C₁₂)-alkylgruppe, eine Formylgruppe, eine C(O)-(C₁-C₁₂)-Allcyl-gruppe, eine substituierte oder unsubstituierte G(O)-Phenylgruppe, eine C(O)NH-(C₁-C₁₂)-Alkylgruppe, eine C(O)FIH-Phenylgruppe, eine substituierte oder unsubstituierte Phenyl-gruppe oder eine Benzylgruppe darstellen;
R2 und R3 gleich oder verschieden sein können und unabhängig voneinander eine gesättigte oder ungesättigte (C₁-C₁₂)-Alkylgruppe darstellen;
R5 und R6 gleich oder verschieden sein können und unabhängig voneinander Wasserstoff, ein Halogenatom (F, CI, Br, J), eine gesättigte oder ungesättigte (C₁-C₁₂)-Alkylgruppe, eine mit einem Halogenatom (F, CI, Br, J) substituierte (C₁-C₁₂)-Alkylgruppe, eine (C₁-C₁₂)-Hydroxyalkylgruppe, eine (C₁-C₁₂)-Alkoxygruppe, eine Cyano-gruppe, eine Nitrogruppe, eine Aminogruppe, eine (C₁-C₁₂)-Alkylamino-gruppe, eine (C₁-C₁₂)-Dialkylaminogruppe, eine Carbonsäure, eine C(O)O-(C₁-C₁₂)-Alkylgruppe, eine substituierte oder unsubstituierte C(O)O-Phenylgruppe, eine substituierte oder unsubstituierte Phenylgruppe oder eine Naphtylgruppe darstellen;
und wenn **Y** und **Z** gleich C-R3 und C-R4 sind, **R5** und **R6** gemeinsam mit dem Restmolekül ein heterozyklisches oder carbozyklisches, gesättigtes oder ungesättigtes, substituiertes oder unsubstituiertes Ringsystem bilden; (b) mindestens eine Kupplersubstanz oder deren physiologisch verträgliches Salz,
sowie (c) als oxidatives Mittel ein Persulfatsalz enthält.

Bevorzugt sind Hydrazon-Derivate der Formel (I) oder deren physiologisch verträgliche Salze, worin **X** gleich Schwefel ist, **Y** und **Z** gleich C-R5 und C-R6 sind, und **R2** und **R3** eine Methylgruppe darstellen; wobei Hydrazon-Derivate der Formel (I) oder deren physiologisch verträgliche Salze, bei denen X gleich Schwefel ist, **R2** und **R3** gleich eine Methylgruppe ist, **R1** gleich einer gesättigten oder ungesättigten (C₁-C₁₂)-Alkylgruppe, einer Hydroxy-(C₁-C₁₂)-alkylgruppe, einer Amino-(C₁-C₁₂)-alkylgruppe, oder einer substituierten oder unsubstituierten Phenylgruppe ist, und **R5** und **R6** unabhängig voneinander Wasserstoff, eine gesättigte oder ungesättigte (C₁-C₁₂)-Alkylgruppe, eine Cyanogruppe, eine Nitrogruppe, eine Aminogruppe, eine (C₁-C₁₂)-Alkylaminogruppe, eine (C₁-C₁₂)-Dialkylaminogruppe, eine C(O)O-(C₁-C₁₂)-Alkylgruppe oder eine substituierte oder unsub-stituierte Phenylgruppe oder eine Naphtylgruppe darstellen, oder R5 und R6 gemeinsam mit dem Restmolekül ein carbozyklisches, ungesättigtes, substituiertes oder unsubstituiertes Ringsystem bilden, besonders bevorzugt sind.

Als Beispiel für die Verbindungen der Formel (1) können die folgenden Verbindungen genannt werden:
3-Methyl-2(3H)-thiazolon-(1-methylethyliden)hydrazon,
3,4-Dimethyl-2(3H)-thiazolon-(1-methylethyliden)hydrazon,
3-Methyl-4-tert-butyl-2(3H)-thiazolon-(1-methylethyliden)hydrazon,
3-Methyl-4-phenyl-2(3H)-thiazolon-(1-methylethyliden)hydrazon,
3-Methyl-4-(4-tolyl)-2(3H)-thiazolon-(1-methylethyliden)hydrazon,
4-(4-Methoxy)phenyl-3-methyl-2(3H)-thiazolon-(1-methylethyliden)hydrazon,
4-(3-Hydroxyphenyl)-3-methyl-2(3H)-thiazolon-(1-methylethyliden)hydrazon,
4-(4-Ethoxyphenyl)-3-methyl-2(3H)-thiazolon-(1-methylethyliden)hydrazon,
4-(4-Bromphenyl)-3-methyl-2(3H)-thiazolon-(1-methylethyliden)hydrazon,
4-(3-Bromphenyl)-3-methyl-2(3H)-thiazolon-(1-methylethyliden)hydrazon,
4-(4-Chlorphenyl)-3-methyl-2(3H)-thiazolon-(1-methylethyliden)hydrazon,
4-(3-Chlorphenyl)-3-methyl-2(3H)-thiazolon-(1-methylethyliden)hydrazon,
3-Methyl-4-(4-nitrophenyl)-2(3H)-thiazolon-(1-methylethyliden)hydrazon,
3-Methyl-4-(3-nitrophenyl)-2(3H)-thiazolon-(1-methylethyliden)hydrazon,
4-([1,1'-Biphenyl]-4-yl)-3-methyl-2(3H)-thiazolon-(1-methylethyliden)hydrazon,
3-Methyl-4-(2-naphthalenyl)-2(3H)-thiazolon-(1-methylethyliden)hydrazon,
2-[(1-Methylethyliden)hydrazono]- 2,3-dihydro-3-methyl-4-thiazolcarbonsäureethylester,
3,4,5-Trimethyl-2(3H)-thiazolon-(1-methylethyliden)hydrazon,
3,4-Dimethyl-5-phenyl-2(3H)-thiazolon-(1-methylethyliden)hydrazon,
3,5-Dimethyl-4-phenyl-2(3H)-thiazolon-(1-methylethyliden)hydrazon,
3-Methyl-4,5-diphenyl-2(3H)-thiazolon-(1-methylethyliden)hydrazon,
5-Ethyl-3-methyl-4-phenyl-2(3H)-thiazolon-(1-mefihylethyliden)hydrazon,
4-(4-Bromphenyl)-3-methyl-5-phenyl-2(3H)-thiazolon-(1-methylethyliden)-hydrazon,
3-Methyl-5-phenyl-4-(4-tolyl)-2(3H)-thiazolon-(1-methylethyliden)hydrazon,
5-(4-Chlorphenyl)-4-phenyl-3-methyl-2(3H)-thiazolon-(1-methylethyliden)-hyd razon,
5-(4-Chlorphenyl)-4-(4-methoxyphenyl)-3-methyl-2(3H)-thiazolon-(1-methylethyliden)hydrazon,
2-[(1-Methylethyliden)hydrazono]-2,3-dihydro-3,4-dimethyl-4-thiazolcarbonsäureethylester,
4-Amino-2-[(1-methylethyliden)hydrazono]-2,3-dihydro-3-methyl-5-thiazolcarbonitril,
3-Ethyl-4,5-dimethyl-2(3H)-thiazolon-(1-methylethyliden)hydrazon,
2-[(1-Methylethyliden)hydrazono]-2,3-dihydro-3-ethyl-4-methyl-thiazolcarbonsäureethylester,
5-Methyl-3-(1-methylethyl)-4-phenyl-2(3H)-thiazolon-(1-methylethyliden)hydrazon,
4,5-Dimethyl-3-(1-methylethyl)-2(3H)-thiazolon-(1-methylethyliden)hydrazon,
3-(1-Methylethyl)-4,5-diphenyl-2(3H)-thiazolon-(1-methylethyliden)hydrazon,
4-Methyl-3-propyl-2(3H)-thiazolon-(1-methylethyliden)hydrazon,
4,5-Dimethyl-3-propyl-2(3H)-thiazolon-(1-methylethyliden)hydrazen,
4,5-Diphenyl-3-propyl-2(3H)-thiazolon-(1-methylethyliden)hydrazon,
3-Butyl-4,5-dimethyl-2(3H)-thiazolon-(1-methylethyliden)hydrazon,
3-Butyl-4,5-diphenyl-2(3H)-thiazolon-(1-methylethyliden)hydrazon,
4,5-Dimethyl-3-(2-methylpropyl)-2(3H)-thiazolon-(1-methylethyliden)-hydrazon,
3-(2-Methylpropyl)-4,5-diphenyl-2(3H)-thiazolon-(1-methytethyliden)-hydrazon,
3-Hydroxyethyl-2(3H)-thiazolon-(1-methylethyliden)hydrazon,
3-Hydroxyethyl-4-methyl-2(3H)-thiazolon-(1-methylethyliden)hydrazon,
3-Hydroxyethyl-4,5-dimethyl-2(3H)-thiazolon-(1-methylethyliden)hydrazon,
3-Aminoethyl-2(3H)-thiazolon-(1-methylethyliden)hydrazon,
3-Aminoethyl-4-methyl-2(3H)-thiazolon-(1-methylethyliden)hydrazon,
3-Aminoethyl-4,5-dimethyl-2(3H)-thiazolon-(1-methylethyliden)hydrazon,
3-Allyl-2(3H)-thiazolon-(1-methylethyliden)hydrazon,
3-Allyl-4-methyl-2(3H)-thiazolon-(1-methylethyliden)hydrazon,
3-Allyl-4-tert-butyl-2(3H)-thiazolon-(1-methylethyliden)hydrazon,
3-Allyl-4-phenyl-2(3H)-thiazolon-(1-methylethyliden)hydrazon,
3-Allyl-4,5-dimethyl-2(3H)-thiazolon-(1-methylethyliden)hydrazon,
3-Allyl-4,5-diphenyl-2(3H)-thiazolon-(1-methylethyliden)hydrazon,
3-Phenyl-2(3H)-thiazolon-(1-methylethyliden)hydrazon,
3,4-Diphenyl-2(3H)-thiazolon-(1-methylethyliden)hydrazon,
4-Methyl-3-phenyl-2(3H)-thiazolon-(1-methylethyliden)hydrazon,
4-p-Biphenylyl-3-phenyl-2(3H)-thiazolon-(1-methylethyliden)hydrazon,
4-(4-Methoxy)phenyl-3-phenyl-2(3H)-thiazolon-(1-methylethyliden)hydrazon,
4-tert-Butyl-3-phenyl-2(3H)-thiazolon-(1-methylethyliden)hydrazon,
2-[(1-Methylethyliden)hydrazono]-2,3-dihydro-3-phenyl-4-thiazolacetessigsäure ethylester,
4,5-Dimethyl-3-phenyl-2(3H)-thiazolon-(1-methylethyliden)hydrazon,
5-Methyl-3,4-diphenyl-2(3H)-thiazolon-(1-methylethyliden)hydrazon,
3,4,5-Triphenyl-2(3H)-thiazolon-(1-methylethyliden)hydrazon,
4,5-Dimethyl-3-(phenylmethyl)-2(3H)-thiazolon-(1-methylethyliden)hydrazon,
4-Phenyl-3-(phenylmethyl)-2(3H)-thiazolon-(1-methylethyliden)hydrazon,
5-Methyl-4-phenyl-3-(phenylmethyl)-2(3H)-thiazolon-(1-methylethyliden)hydrazon,
2-[(1-Methylethyliden)hydrazono]-2,3-dihydro-3-[(phenylamino)carbonyl]-4-methyl-thiazolcarbonsäureethylester,
3-Methyl-4,5,6,7-tetrahydro-2(3H)-benzothiazolon-(1-methylethyliden)-hydrazon,
3-Methyl-2(3H)-benzothiazolon-(1-methylethyliden)hydrazon,
3,6-Dimethyl-2(3H)-benzothiazolon-(1-methylethyliden)hydrazon,
6-Chlor-3-methyl-2(3H)-benzothiazolon-(1-methylethyliden)hydrazon,
7-Chlor-3-methyl-2(3H)-benzothiazolon-(1-methylethyliden)hydrazon,
6-Hydroxy-3-methyl-2(3H)-benzothiazolon-(1-methylethyliden)hydrazon,
5-Methoxy-3-methyl-2(3H)-benzothiazolon-(1-methylethyliden)hydrazon,
7-Methoxy-3-methyl-2(3H)-benzothiazolon-(1-methylethyliden)hydrazon,
5,6-Dimethoxy-3-methyl-2(3H)-benzothiazolon-(1-methylethyliden)-hydrazon,
5-Ethoxy-3-methyl-2(3H)-benzothiazolon-(1-methylethyliden)hydrazon,
6-Ethoxy-3-methyl-2(3H)-benzothiazolon-(1-methylethyliden)hydrazon,
3-Methyl-5-nitro-2(3H)-benzothiazolon-(1-methylethyliden)hydrazon,
3-Methyl-6-nitro-2(3H)-benzothiazolon-(1-methylethyliden)hydrazon,
5-Acetamido-3-methyl-2(3H)-benzothiazolon-(1-methylethyliden)hydrazon,
6-Acetamido-3-methyl-2(3H)-benzothiazolon-(1-methylethyliden)hydrazon,
5-Anilino-3-methyl-2(3H)-benzothiazolon-(1-methylethyliden)hydrazon,
6-Anilino-3-methyl-2(3H)-benzothiazolon-(1-methylethyliden)hydrazon,
2-[(1-Methylethyliden)hydrazono]-2,3-dihydro-3-methyl-6-benzothiazolcarbonsäure,
2-[(1-Methylethyliden)hydrazono]-2,3-dihydro-3-methyl-4-benzothiazolsulfonsäure,
2-[(1-Methylethyliden)hydrazono]-2,3-dihydro-3-methyl-5-benzothiazol-sulfonsäure,
2-[(1-Methylethyliden)hydrazono]-2,3-dihydro-3-methyl-6-benzothiazol-sulfonsäure,
2-[(1-Methylethyliden)hydrazono]-2,3-dihydro-3-methyl-7-benzothiazol-sulfonsäure,
2-[(1-Methylethyliden)hydrazono]-2,3-dihydro-N,N,3-trimethyl-6-benzothiazol-sulfonsäureamid,
[(2-[(1-Methylethyliden)hydrazono]-2,3-dihydro-3-methyl-6-benzothiazolyl)-oxy]essigsäure-hydrazid,
3-Methyl-naphtho[2,3-d]thiazol-2(3H)-on-(1-methylethyliden)hydrazon,
3-Ethyl-2(3H)-benzothiazolon-(1-methylethyliden)hydrazon,
6-Ethoxy-3-ethyl-2(3H)-benzothiazolon-(1-methylethyliden)hydrazon,
3-Propyl-2(3H)-benzothiazolon-(1-methylethyliden)hydrazon,
3-Butyl-2(3H)-benzothiazolon-(1-methylethyliden)hydrazon,
3-Hexyl-2(3H)-benzothiazolon-(1-methylethyliden)hydrazon,
3-Hydroxyethyl-2(3H)-benzothiazolon-(1-methylethyliden)hydrazon,
3-Aminoethyl-2(3H)-benzothiazolon-(1-methylethyliden)hydrazon,
3-(4-Methylbenzyl)-2(3H)-benzothiazolon-(1-methylethyliden)hydrazon,
2-[(1-Methylethyliden)hydrazono]-2,3-dihydro-3-(2-hydroxyethyl)-6-benzothiazol-carbonsäure,
2-[(1-Methylethyliden)hydrazono]-2,3-dihydro-6-methoxy-3(2H)-benzothiazol-propan-sulfonsäure,
6-Hexadecyloxy-2-[(1-methylethyliden)hydrazono]-3(2H)-benzothiazol-propan-sulfonsäure,
2-Oxo-3-benzothiazolin-essigsäureethylester-(1-methylethyliden)hydrazon,
3-Acetyl-2(3H)-benzothiazolon-(1-methylethyliden)hydrazon,
2-[(1-Methylethyliden)hydrazono]-3(2H)-benzothiazol-carboxaldehyd,
3-Methyl-2(3H)-oxazolon-(1-methylethyliden)hydrazon,
3-Phenyl-2(3H)-oxazolon-(1-methylethyliden)hydrazon,
3-Methyl-2(3H)-benzoxazolon-(1-methylethyliden)hydrazon,
3-Phenyl-2(3H)-benzoxazolon-(1-methylethyliden)hydrazon,
1,3-Dimethyl-4-imidazolin-2-on-(1-methylethyliden)hydrazon,
1,3-Diethyl-4-imidazolin-2-on-(1-methylethyliden)hydrazon,
1,3-Dihydroxyethyl-4-imidazolin-2-on(1-methylethyliden)hydrazon,
1,3-Diaminoethyl-4-imidazolin-2-on-(1-methylethyliden)hydrazon,
1,3-Dimethyl-4-methoxy-4-imidazolin-2-on-(1-methylethyliden)hydrazon,
1,3,4-Trimethyl-4-imidazolin-2-on-(1-methylethyliden)hydrazon,
1,3-Dimethyl-4-phenyl-4-imidazolin-2-on-(1-methylethyliden)hydrazon,
4-Carboxy-1,3-dimethyl-4-imidazolin-2-on-(1-methylethyliden)hydrazon,
4-Amino-1,3-dimethyl-4-imidazolin-2-on-(1-methylethyliden)hydrazon,
1,3-Dimethyl-4-dimethylamino-4-imidazolin-2-on-(1-methylethyliden)-hydrazon,
1,3-Dimethyl-2-benzimidazolinon-(1-methylethyliden)hydrazon,
1,3-Diethyl-2-benzimidazolinon-(1-methylethyliden)hydrazon,
1,3-Dihydroxyethyl-2-benzimidazolinon-(1-methylethyliden)hydrazon,
1,3-Diaminoethyl-2-benzimidazolinon-(1-methylethyliden)hydrazon,
1,3,5-Trimethyl-2-benzimidazolinon-(1-methylethyliden)hydrazon,
5-Methoxy-1,3-dimethyl-2-benzimidazolinon-(1-methylethyliden)hydrazon,
5-Brom-1,3-dimethyl-2-benzimidazolinon-(1-methylethyliden)hydrazon,
4,6-Dibrom-1,3-dimethyl-2-benzimidazolinon-(1-methylethyliden)hydrazon,
5-Chlor-1,3-dimethyl-2-benzimidazolinon-(1-methylethyliden)hydrazon,
1,3-Dimethyl-5-nitro-2-benzimidazolinon-(1-methylethyliden)hydrazon,
1,3-Dimethyl-6-nitro-2-benzimidazolinon-(1-methylethyliden)hydrazon,
1,4-Dimethyl-Δ2-1,2,4-triazolin-5-on-(1-methylethyliden)hydrazon,
1,4-Dihydroxyethyl-Δ2-1,2,4-triazolin-5-on-(1-methylethyliden)hydrazon,
1,4-Diaminoethyl-Δ2-1,2,4-triazolin-5-on-(1-methylethyliden)hydrazon,
1,3,4-Trimethyl-Δ2-1,2,4-triazolin-5-on-(1-methylethyliden)hydrazon,
1,4-Dimethyl-3-phenyl-Δ2-1,2,4-triazolin-5-on-(1-methylethyliden)hydrazon,
1,4-Dimethyl-3-methoy-Δ2-1,2,4-triazolin-5-on-(1-methylethyliden)hydrazon,
1,4-Dimethyl-3-dimethylamino-Δ2-1,2,4-triazolin-5-on-(1-methylethyliden)-hydrazon,
4-Carboxy-1,4-dimethyl-Δ2-1,2,4-triazolin-5-on-(1-methylethyliden)hydrazon,
4-Amino-1,4-dimethyl-Δ2-1,2,4-triazolin-5-on-(1-methylethyliden)hydrazon,
4-Butyl-1-methyl-3-phenyl-Δ2-1,3,4-triazolin-5-on-(1-methylethyliden)-hydrazon,
4-Methyl-Δ2-1,3,4-thiadiazolin-5-on-(1-methylethyliden)hydrazon,
4-Hydroxyethyl-Δ2-1,3,4-thiadiazolin-5-on-(1-methylethyliden)hydrazon,
4-Aminoethyl-Δ2-1,3,4-thiadiazolin-5-on-(1-methylethyliden)hydrazon,
4-Methyl-2-phenyl-Δ2-1,3,4-thiadiazolin-5-on-(1-methylethyliden)hydrazon,
2-Methoxy-4-methyl-Δ2-1,3,4-thiadiazolin-5-on-(1-methylethyliden)hydrazon,
2-Anilino-4-methyl-Δ2-1,3,4-thiadiazolin-5-on-(1-methylethyliden)hydrazon,
2-Amino-4-methyl-Δ2-1,3,4-thiadiazolin-5-on-(1-methylethyliden)hydrazon,
2-Dimethylamino-4-methyl-Δ2-1,3,4-thiadiazolin-5-on-(1-methylethyliden)-hydrazon,
4-Methyl-2-(methylthio)-Δ2-1,3,4-thiadiazolin-5-on-(1-methylethyliden)-hydrazon,
4-(5-[(1-Methylethyliden)hydrazono]-4,5-dihydro-4-methyl-Δ2-1,3,4-thiadiazol-2-yl)-benzensulfonyl-fluorid,
4-Methyl-Δ2-1,2,4-thiadiazolin-5-on-(1-methylethyliden)hydrazon,
4-Hydroxyethyl-Δ2-1,2,4-thiadiazolin-5-on-(1-methylethyliden)hydrazon,
4-Aminoethyl-Δ2-1,2,4-thiadiazolin-5-on-(1-methylethyliden)hydrazon,
4-Methyl-3-phenyl-Δ2-1,2,4-thiadiazolin-5-on-(1-methylethyliden)hydrazon,
3-Methoxy-4-methyl-Δ2-1,2,4-thiadiazolin-5-on-(1-methylethyliden)hydrazon,
3-Amino-4-methyl-Δ2-1,2,4-thiadiazolin-5-on-(1-methylethyliden)hydrazon,
3-Dimethylamino-4-methyl-Δ2-1,2,4-thiadiazolin-5-on-(1-methylethyliden)-hydrazon,
3-Carboxy-4-methyl-Δ2-1,2,4-thiadiazolin-5-on-(1-methylethyliden)hydrazon,
1,4-Dimethyl-Δ2-1,2,4-triazolin-5-on-(1-methylethyliden)hydrazon,
1,4-Dihydroxyethyl-Δ2-1,2,4-triazolin-5-on-(1-methylethyliden)hydrazon,
1,4-Aminoethyl-Δ2-1,2,4-triazolin-5-on-(1-methylethyliden)hydrazon,
1,3,4-Trimethyl-Δ2-1,2,4-triazolin-5-on-(1-methylethyliden)hydrazon,
1,4-Dimethyl-3-phenyl-Δ2-1,2,4-triazolin-5-on-(1-methylethyliden)hydrazon
und 4-Methyl-3-phenyl-Δ2-1,2,4-triazolin-5-on-(1-methylethyliden)hydrazon.

Besonders bevorzugt sind die folgenden Verbindungen der Formel (I):
3-Methyl-2(3H)-thiazolon-(1-methylethyliden)hydrazon,
3,4-Dimethyl-2(3H)-thiazolon-(1-methylethyliden)hydrazon,
4-tert-Butyl-3-methyl-2(3H)-thiazolon-(1-methylethyliden)hydrazon,
3-Methyl-4-phenyl-2(3H)-thiazolon-(1-methylethyliden)hydrazon,
3-Methyl-4-(4-tolyl)-2(3H)-thiazolon-(1-methylethyliden)hydrazon,
4-(4-Methoxy)phenyl-3-methyl-2(3H)-thiazolon-(1-methylethyliden)hydrazon,
4-(3-Hydroxyphenyl)-3-methyl-2(3H)-thiazolon-(1-methylethyliden)hydrazon,
4-(4-Ethoxyphenyl)-3-methyl-2(3H)-thiazolon-(1-methylethyliden)hydrazon,
4-(4-Bromphenyl)-3-methyl-2(3H)-thiazolon-(1-methylethyliden)hydrazon,
4-(3-Bromphenyl)-3-methyl-2(3H)-thiazolon-(1-methylethyliden)hydrazon,
4-(4-Chlorphenyl)-3-methyl-2(3H)-thiazolon-(1-methylethyliden)hydrazon,
4-(3-Chlorphenyl)-3-methyl-2(3H)-thiazolon-(1-methylethyliden)hydrazon,
3-Methyl-4-(4-nitrophenyl)-2(3H)-thiazolon-(1-methylethyliden)hydrazon,
3-Methyl-4-(3-nitrophenyl)-2(3H)-thiazolon-(1-methylethyliden)hydrazon,
4-([1,1'-Biphenyl]-4-yl)-3-methyl-2(3H)-thiazolon-(1-methylethyliden)-hydrazon,
2-[(1-Methylethyliden)hydrazono]- 2,3-dihydro-3-methyl-4-thiazolcarbonsäureethytester,
3,4,5-Trimethyl-2(3H)-thiazolon-(1-methylethyliden)hydrazon,
3,4-Dimethyl-5-phenyl-2(3H)-thiazolon-(1-methylethyliden)hydrazon,
3,5-Dimethyl-4-phenyl-2(3H)-thiazolon-(1-methylethyliden)hydrazon,
3-Methyl-4,5-diphenyl-2(3H)-thiazolon-(1-methylethyliden)hydrazon,
5-Ethyl-3-methyl-4-phenyl-2(3H)-thiazolon-(1-methylethyliden)hydrazon,
4-(4-Bromphenyl)-3-methyl-5-phenyl-2(3H)-thiazolon-(1-methylethyliden)-hydrazon,
3-Methyl-5-phenyl-4-(4-tolyl)-2(3H)-thiazolon-(1-methylethyliden)hydrazon,
5-(4-Chlorphenyl)-4-phenyl-3-methyl-2(3H)-thiazolon-(1-methylethyliden)-hydrazon,
5-(4-Chlorphenyl)-4-(4-methoxyphenyl)-3-methyl-2(3H)-thiazolon-(1-methylethyliden)hydrazon,
2-[(1-Methylethyliden)hydrazono]-2,3-dihydro-3,4-dimethyl-4-thiazolcarbonsäureethylester,
4-Amino-2-[(1-methylethyliden)hydrazono]-2,3-dihydro-3-methyl-5-thiazolcarbonitril,
3-Ethyl-4,5-dimethyl-2(3H)-thiazolon-(1-methylethyliden)hydrazon,
2-[(1-Methylethyliden)hydrazono]-2,3-dihydro-3-ethyl-4-methyl-thiazolcarbonsäureethylester,
5-Methyl-3-(1-methylethyl)-4-phenyl-2(3H)-thiazolon-(1-methylethyliden)-hydrazon,
4,5-Dimethyl-3-(1-methylethyl)- 2(3H)-thiazolon-(1-methylethyliden)-hydrazon,
3-(1-Methylethyl)-4,5-diphenyl-2(3H)-thiazolon-(1-methylethyliden)-hydrazon,
4-Methyl-3-propyl-2(3H)-thiazolon-(1-methylethyliden)hydrazon,
4,5-Dimethyl-3-propyl-2(3H)-thiazolon-(1-methylethyliden)hydrazon,
4,5-Diphenyl-3-propyl-2(3H)-thiazolon-(1-methylethyliden)hydrazon,
3-Butyl-4,5-dimethyl-2(3H)-thiazolon-(1-methylethyliden)hydrazon,
3-Butyl-4,5-diphenyl-2(3H)-thiazolon-(1-methylethyliden)hydrazon,
4,5-Dimethyl-3-(2-methylpropyl)- 2(3H)-thiazolon-(1-methylethyliden)-hydrazon,
3-(2-Methylpropyl)- 4,5-diphenyl-2(3H)-thiazolon-(1-methylethyliden)-hydrazon,
3-Allyl-2(3H)-thiazolon-(1-methylethyliden)hydrazon,
3-Allyl-4-methyl-2(3H)-thiazolon-(1-methylethyliden)hydrazon,
3-Allyl-4-tert-butyl-2(3H)-thiazolon-(1-methylethyliden)hydrazon,
3-Allyl-4-phenyl-2(3H)-thiazolon-(1-methylethyliden)hydrazon,
3-Allyl-4,5-dimethyl-2(3H)-thiazolon-(1-methylethyliden)hydrazon,
3-Allyl-4,5-diphenyl-2(3H)-thiazolon-(1-methylethyliden)hydrazon,
3-Hydroxyethyl-2(3H)-thiazolon-(1-methylethyliden)hydrazon,
3-Hydroxyethyl-4-methyl-2(3H)-thiazolon-(1-methylethyliden)hydrazon,
3-Hydroxyethyl-4,5-dimethyl-2(3H)-thiazolon-(1-methylethyliden)hydrazon,
3-Aminoethyl-2(3H)-thiazolon-(1-methylethyliden)hydrazon,
3-Aminoethyl-4-methyl-2(3H)-thiazolon-(1-methylethyliden)hydrazon,
3-Aminoethyl-4,5-dimethyl-2(3H)-thiazolon-(1-methylethyliden)hydrazon,
3-Phenyl-2(3H)-thiazolon-(1-methylethyliden)hydrazon,
3,4-Diphenyl-2(3H)-thiazolon-(1-methylethyliden)hydrazon,
4-Methyl-3-phenyl-2(3H)-thiazolon-(1-methylethyliden)hydrazon,
4-p-Biphenylyl-3-phenyl-2(3H)-thiazolon-(1-methylethyliden)hydrazon,
4-(4-Methoxy)phenyl-3-phenyl-2(3H)-thiazolon-(1-methylethyliden)-hydrazon,
4-tert-Butyl-3-phenyl-2(3H)-thiazolon-(1-methylethyliden)hydrazon,
4,5-Dimethyl-3-phenyl-2(3H)-thiazolon-(1-methylethyliden)hydrazon,
5-Methyl-3,4-diphenyl-2(3H)-thiazolon-(1-methylethyliden)hydrazon,
3,4,5-Triphenyl-2(3H)-thiazolon-(1-methylethyliden)hydrazon,
4,5-Dimethyl-3-(phenylmethyl)-2(3H)-thiazolon-(1-methylethyliden)hydrazon,
4-Phenyl-3-(phenylmethyl)-2(3H)-thiazolon-(1-methylethyliden)hydrazon,
5-Methyl-4-phenyl-3-(phenylmethyl)-2(3H)-thiazolon-(1-methylethyliden)-hydrazon,
3-Methyl-4,5,6,7-tetrahydro-2(3H)-benzothiazolon-(1-methylethyliden)-hydrazon,
3-Methyl-2(3H)-benzothiazolon-(1-methylethyliden)hydrazon,
3,6-Dimethyl-2(3H)-benzothiazolon-(1-methylethyliden)hydrazon,
6-Chlor-3-methyl-2(3H)-benzothiazolon-(1-methylethyliden)hydrazon,
7-Chlor-3-methyl-2(3H)-benzothiazolon-(1-methylethyliden)hydrazon,
6-Hydroxy-3-methyl-2(3H)-benzothiazolon-(1-methylethyliden)hydrazon,
5-Methoxy-3-methyl-2(3H)-benzothiazolon-(1-methylethyliden)hydrazon,
7-Methoxy-3-methyl-2(3H)-benzothiazolon-(1-methylethyliden)hydrazon,
5,6-Dimethoxy-3-methyl-2(3H)-benzothiazolon-(1-methylethyliden)hydrazon,
5-Ethoxy-3-methyl-2(3H)-benzothiazolon-(1-methylethyliden)hydrazon,
6-Ethoxy-3-methyl-2(3H)-benzothiazolon-(1-methylethyliden)hydrazon,
3-Methyl-5-nitro-2(3H)-benzothiazolon-(1-methylethyliden)hydrazon,
3-Methyl-6-nitro-2(3H)-benzothiazolon-(1-methylethyliden)hydrazon,
5-Acetamido-3-methyl-2(3H)-benzothiazolon-(1-methylethyliden)hydrazon,
6-Acetamido-3-methyl-2(3H)-benzothiazolon-(1-methylethyliden)hydrazon,
5-Anilino-3-methyl-2(3H)-benzothiazolon-(1-methylethyliden)hydrazon,
6-Anilino-3-methyl-2(3H)-benzothiazolon-(1-methylethyliden)hydrazon,
2-[(1-Methylethyliden)hydrazono]-2,3-dihydro-3-methyl-6-benzothiazolcarbonsäure,
2-[(1-Methylethyliden)hydrazono]-2,3-dihydro-3-methyl-4-benzothiazolsulfonsäure,
2-[(1-Methylethyliden)hydrazono]-2,3-dihydro-3-methyl-5-benzothiazolsulfonsäure,
2-[(1-Methylethyliden)hydrazono]-2,3-dihydro-3-methyl-6-benzothiazolsulfonsäure,
2-[(1-Methylethyliden)hydrazono]-2,3-dihydro-3-methyl-7-benzothiazolsulfonsäure,
2-[(1-Methylethyliden)hydrazono]-2,3-dihydro-N,N-3-trimeihyl-6-benzothiazol-sulfonsäureamid,
[(2-[(1-Methylethyliden)hydrazono]-2,3-dihydro-3-methyl-6-benzothiazolyl)oxy]essigsäure-hydrazid,
3-Ethyl-2(3H)-benzothiazolon-(1-methylethyliden)hydrazon,
6-Ethoxy-3-ethyl-2(3H)-benzothiazolon-(1-methylethyliden)hydrazon,
3-Propyl-2(3H)-benzothiazolon-(1-methylethyliden)hydrazon,
3-Butyl-2(3H)-benzothiazolon-(1-methylethyliden)hydrazon und
3-Hexyl-2(3H)-benzothiazolon-(1-methylethyliden)hydrazon.

Die Verbindungen der Formel (I) können nach aus der Literatur bekannten Syntheseverfahren, beispielsweise der Vorschrift in Research Disclosure October 1978, Seite 42 - 44 (1978), No. 17434 beschriebenen Verfahren hergestellt werden.

Als Kupplersubstanzen kommen insbesondere die folgenden Kupplersubstanzen oder deren Salze in Betracht:
N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxy-ethoxy)-5-methyl-benzol, 2,4-Di[(2-hydroxyethyl)amino]-1,5-dimethoxy-benzol, 2,3-Diamino-6-methoxypyridin, 3-Amino-6-methoxy-2-(methyl-amino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-meihylethyl)-phenol, 3-[(2-Hydroxyethyl)-amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methylphenol, 5-Amino-4-fluor-2-methyl-phenol, 5-Amino-4-methoxy-2-methylphenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methylphenol, 3-[(2-Hydroxyethyl)-aminol-2-methyl-phenol, 2-Amino-3-hydroxy-pyridin, 2,6-Dihydroxy-3,4-dimethylpyridin, 5-Amino-4-chlor-2-methylphenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzo-dioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion in Betracht.

Als Persulfatsalze kommen zum Beispiel Kaliumpersulfat, Natriumpersulfat oder Ammoniumpersulfat sowie deren Mischungen in Betracht.

Die Persulfatsalze sind in dem gebrauchsfertigen Färbemittel (A) in einer Gesamtmenge von etwa 0,01 bis 10 Gewichtsprozent, vorzugsweise etwa 0,1 bis 5 Gewichtsprozent, enthalten.

Weiterhin kann das erfindungsgemäße Färbemittel zusätzlich zu den Verbindungen der Formel (I) sowie den Kupplersubstanzen gegebenenfalls zusätzlich weitere übliche, physiologisch unbedenkliche, direktziehende Farbstoffe aus der Gruppe der kationischen und anionischen Farbstoffe, der Dispersionsfarbstoffe, der Azofarbstoffe, der Chinonfarbstoffe und der Triphenylmethanfarbstoffe enthalten.

Die direktziehenden Farbstoffe sind in dem gebrauchsfertigen Färbemittel (A) in einer Menge von etwa 0,01 bis 10 Gewichtsprozent, vorzugsweise etwa 0,1 bis 5 Gewichtsprozent, enthalten.

Weiterhin kann das erfindungsgemäße Färbemittel zusätzlich zu den Verbindungen der Formel (I) gegebenenfalls weitere übliche Entwicklersubstanzen enthalten, wie zum Beispiel 1,4-Diamino-benzol (p-Phenylendiamin), 1,4-Diamino-2-methyl-benzol (p-Toluylendiamin), 1,4-Diamino-2-(thiophen-2-yl)benzol, 1,4-Diamino-2-(thiophen-3-yl)benzol, 4-(2,5-Diaminophenyl)-2-((diethylamino)methyl)-thiophen, 2-Chlor-3-(2,5-diaminophenyl)-thiophen, 1,4-Diamino-2-(pyridin-3-yl)benzol, 2,5-Diamino-biphenyl, 2,5-Diamino-4'-(1-methylethyl)-1,1'-biphenyl, 2,3',5-Triamino-1,1'-biphenyl, 1,4-Diamino-2-methoxymethyl-benzol, 1,4-Diamino-2-aminomethyl-benzol, 1,4-Diamino-2-((phenylamino)methyl)benzol, 1,4-Diamino-2-((ezhyl-(2-hydroxyethyl)-amino)methyl)benzol, 1,4-Diamino-2-hydroxymethyl-benzol, 4-[Di(2-hydroxyethyl)amino]-anilin, 4-(((4-Amino-phenyl)-methyl)amino)-anilin, 4-[(4-Amino-phenylamino)-methyl]-phenol, 1,4-Diamino-N-(4-pyrrolidin-1-yl-benzyl)-benzol, 1,4-Diamino-N-furan-3-ylmethyl-benzol, 1,4-Diamino-N-thiophen-2-ylmethyl-benzol, 1,4-Diamino-N-furan-2-ylmethylbenzol, 1,4-Diamino-N-thiophen-3-ylmethyl-benzol, 1,4-Diamino-N-benzylbenzol, 1,4-Diamino-2-(1-hydroxyethyl)-benzol, 1,4-Diamino-2-(2-hydroxyethyl)-benzol, 1,3-Bis[(4-aminophenyl)(2-hydroxyethyl)amino]-2-propanol, 1,8-Bis(2,5-diaminophenoxy)-3,6-dioxaoctan, 2,5-Diamino-4'-hydroxy-1,1'-biphenyl, 2,5-Diamino-2'-trifluormethyl-1,1-'biphenyl, 2,4',5-Triamino-1,1'-biphenyl, 4-Amino-phenol, 4-Amino-3-methyl-phenol, 4-Methylaminophenol, 4-Amino-2-(amino-methyl)-phenol, 4-Amino-2-[(2-hydroxyethyl)-amino]methyl-phenol, 4-Amino-2-(methoxymethyl)-phenol, 5-Aminosalicylsäure, 2,4,5,6-Tetra-amino-pyrimidin, 2,5,6-Triamino-4-(1H)-pyrimidon, 4,5-Diamino-1-(2-hydroxyethyl)-1H-pyrazol, 4,5-Diamino-1-pentyl-1H-pyrazol, 4,5-Diamino-1-(phenylmethyl)-1H-pyrazol, 4,5-Diamino-1-((4-methoxyphenyl)methyl-1 H-pyrazol, 2-Amino-phenol, 2-Amino-6-methyl-phenol, 2-Amino-5-methyl-phenol, 1,2,4-Trihydroxy-benzol, 2,4-Diaminophenol, 1,4-Dihydroxybenzol oder 2-(((4-Aminophenyl)-amino)methyl)-1,4-diamino-benzol.

Die Verbindungen der Formel (I) sowie die Kupplersubstanzen und die zusätzlichen Entwicklersubstanzen sind in dem gebrauchsfertigen Färbemittel (A) jeweils in einer Gesamtmenge von etwa 0,01 bis 10 Gewichtsprozent, vorzugsweise etwa 0,1 bis 5 Gewichtsprozent, enthalten.

Die Verbindungen der Formel (I) und die Kupplersubstanzen werden in der Regel getrennt voneinander aufbewahrt und erst kurz vor der Anwendung miteinander vermischt und mit dem Persulfatsalz versetzt. Es ist jedoch auch möglich, sofern die Verbindungen der Formel (I), die Kupplersubstanzen und das Persulfatsalz in fester Form vorliegen, diese gemeinsam abzupacken und das gebrauchsfertige Färbemittel (A) kurz vor der Anwendung durch Vermischen der Verbindungen der Formel (I), der Kupplersubstanzen und des Persulfatsalzes mit Wasser oder einer die übrigen Bestandteile des Mittels enthaltenden flüssigen Zubereitung herzustellen.

Das erfindungsgemäße Färbemittel besteht somit in der Regel aus mehreren Komponenten, welche vor der Anwendung miteinander vermischt werden. Vorzugsweise liegt das Mittel in Form eines 2-Komponenten-Kits, bestehend aus einer Farbträgermasse (A1), welche die Verbindung der Formel (1) enthält, und einer weiteren Farbträgermasse (A2), welche die Kupplersubstanzen und die Persulfatsalze enthält, oder eines 3-Komponenten-Kits, bestehend aus einer Farbträgermasse (A1), welche die Verbindung der Formel (I) enthält, einer weiteren Farbträgermasse (A2), welche die Kupplersubstanzen enthält, und einer die Persulfatsalze enthaltenden 3. Komponente (A3), vor.

Ein weiterer Gegenstand der vorliegenden Erfindung ist ein Mehrkomponenten-Kit, bestehend aus einem Mittel der Komponente (A1) und einem Mittel der Komponente (A2), wobei das Persulfat auch getrennt von der Komponente (A2) als Komponente (A3) abgepackt sein kann, sowie gegebenenfalls einem Mittel zur Einstellung des pH-Wertes (Alkalisierungsmittel oder Säure). Selbstverständlich können auch die Mittel der Komponenten (A1) und (A2) aus mehreren Einzelkomponenten bestehen, welche erst unmittelbar vor der Anwendung miteinander vermischt werden. Ebenfalls ist ein 2-Komponenten-Kit möglich, dessen 1. Komponente aus einem die Verbindungen der Formel (I), die Kuppler und die Persulfatsalze sowie gegebenenfalls weitere übliche pulverförmige kosmetische Zusatzstoffe enthaltenden Pulver besteht, und dessen 2. Komponente Wasser oder eine flüssige kosmetische Zubereitung ist. Besonders bevorzugt ist jedoch ein 2-Komponenten-Kit, bestehend aus einem Mittel der Komponente (A1) und einem Mittel der Komponente (A2).

Die vorgenannten direktziehenden Farbstoffe können in der Komponente (A1) oder/und (A2) in einer Gesamtmenge von etwa 0,02 bis 20 Gewichtsprozent, vorzugsweise 0,2 bis 10 Gewichtsprozent, eingesetzt werden, während die Kupplersubstanzen und zusätzlichen Entwicklersubstanzen in der jeweiligen Farbträgermasse (Komponente (A1) bzw. Komponente (A2)) jeweils in einer Gesamtmenge von etwa 0,02 bis 20 Gewichtsprozent, vorzugsweise etwa 0,2 bis 10 Gewichtsprozent, enthalten sein können.

Die Zubereitungsform für die Komponenten (A1) und (A2) sowie das gebrauchsfertige Färbemittel (A) kann beispielsweise eine Lösung, insbesondere eine wässrige oder wässrig-alkoholische Lösung, eine Creme, ein Gel oder eine Emulsion sein. Ihre Zusammensetzung stellt eine Mischung der Verbindung der Formel (I) beziehungsweise der Kupplersubstanzen mit den für solche Zubereitungen üblichen Zusätzen dar.

Übliche in Färbemitteln verwendete Zusätze in Lösungen, Cremes, Emulsionen, Gelen oder Aerosolschäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen obenlächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethytierte Fettalkohle, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (jeweils bezogen auf die Komponente (A1) bzw. (A2)), die Verdicker in einer Menge von etwa 0,1 bis 25 Gewichtsprozent (jeweils bezogen auf die Komponente (A1) bzw. (A2)) und die Pflegestoffe in einer Konzentration von etwa 0,1 bis 5,0 Gewichtsprozent (jeweils bezogen auf die Komponente (A1) bzw. (A2)).

Der pH-Wert des gebrauchsfertigen Färbemittels (A) sowie der Farbträgermassen (A1) und (A2) beträgt jeweils etwa 3 bis 12, vorzugsweise etwa 6 bis 10, wobei sich der pH-Wert des gebrauchsfertigen Färbemittels (A) in der Regel bei der Mischung der Komponente (A1) mit der Komponente (A2) und ggfs. den Persulfaten einstellt. Zur Einstellung des für die Färbung gewünschten pH-Wertes der Komponenten (A1) und (A2) sowie des gebrauchsfertigen Färbemittels (A) können -falls erforderlichjedoch auch alkalisierende Mittel, wie zum Beispiel Ammoniak, Alkalihydroxide, Erdalkalihydroxide, Alkaliacetate, Erdalkaliacetate, Alkalicarbonate oder Erdalkalicarbonate, oder Säuren, wie zum Beispiel Milchsäure, Essigsäure, Weinsäure, Phosphorsäure, Salzsäure, Zitronensäure, Ascorbinsäure oder Borsäure, zugesetzt werden.

Das gebrauchsfertige Färbemittel wird unmittelbar vor der Anwendung durch Vermischen der Komponenten (A1) und (A2) bzw. (A1) und (A2) und (A3) -gegebenenfalls unter Zusatz eines Alkalisierungsmittel oder einer Säure- hergestellt und sodann auf die Faser, insbesondere menschliche Haare, aufgetragen. Je nach gewünschter Farbtiefe läßt man diese Mischung etwa 5 bis 60 Minuten, vorzugsweise etwa 15 bis 30 Minuten, bei einer Temperatur von etwa 20 bis 50 °C, insbesondere bei etwa 30 bis 40 °C einwirken. Anschließend wird die Faser mit Wasser gespült, gegebenenfalls mit einem Shampoo gewaschen und sodann getrocknet.

Das erfindungsgemäße Färbemittel ermöglicht eine gleichmäßige und dauerhafte Färbung der Fasern, insbesondere von Keratinfasern, wie zum Beispiel menschlichen Haaren, wobei eine breite Farbpalette von gelben bis blauen Farbtönen möglich ist. Die Beständigkeit gegenüber Schweiss wird in besonders hohe Masse erfüllt.

Die nachfolgenden Beispiele sollen den Gegenstand näher erläutern, ohne ihn auf diese Beispiele zu beschränken.

### Beispiele

### Beispiel 1: Synthese von 3,4-Dimethyl-2(3H)-thiazolon-1-(methylethyliden)-hydrazon

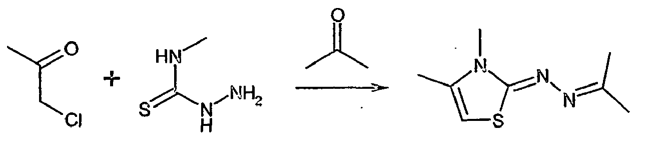

21 g (200 mmol) 4-Methyl-3-thiosemicarbazid werden in 1000 ml Aceton 2 Stunden lang unter Rückfluss gekocht. Dann wird die Lösung tropfen-weise mit 20,4 g (220 mmol) Chloraceton versetzt. Die Reaktionsmischung wird sodann 7 Stunden lang unter Rückfluss gekocht, und anschließend eingeengt. Das so erhaltene Rohprodukt wird aus Aceton umkristallisiert. Es werden 23 g eines orangen Pulvers (63% der Theorie) erhalten.
Schmelzpunkt: 139 - 139,6 °C
¹H-NMR (DMSO, 300 MHz): δ = 6,72 (s, breit, 1 H, H-C(5)); δ = 3,67 (s, 3H, N-CH3); δ = 2,27 (d, J=0,9Hz, 3H, CH3-C(4)); δ = 2,17 (s, 3H, CH3); δ = 2,07 (s, 3H, CH3)
¹³C-NMR (DMSO, 300 MHz): δ = 169,16; δ = 164,14; δ = 139,02 (C(4));
δ = 103,36 (C(5)); δ = 34,47 (CH₃N); δ = 24,60; δ = 19,91; δ = 13,53 (CH₃-C(4)). MS (ESI): 184 [M+H]⁺ (100)

### Beispiele 2-9: Synthese von Derivaten der Formel (I) (allgemeine Synthese Vorschrift)

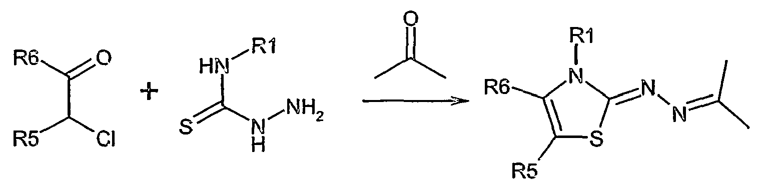

4 mmol substituiertes Thiosemicarbazid werden in 20 ml Aceton 2 Stunden lang unter Rückfluss gekocht. Dann wird die Lösung tropfen-weise mit 4,4 mmol α-Chlor-keton versetzt. Die Reaktionsmischung wird sodann 7 Stunden lang unter Rückfluss gekocht, und anschließend eingeengt. Das so erhaltene 2(3H)-Thiazolon-1-(methylethyliden)-hydrazon wird aus Aceton umkristallisiert.

### 2. 3-Methyl-4-phenyl-2(3H)-thiazolon-1-(methylethyliden)-hydrazon

Aus 4-Methyl-3-thiosemicarbazid und Phenacylchlorid
Ausbeute: 1 g (100 % der Theorie)
ESI-MS: 246 [M+H]⁺ (100)

### 3. 4-tert-Butyl-3-methyl-2(3H)-thiazolon-1-(methylethyliden)-hydrazon

Aus 4-Methyl-3-thiosemicarbazid und 1-Chlor-3,3-dimethyl-2-butanon
Ausbeute: 0,67 g (75 % der Theorie)
ESI-MS: 226 [M+H]⁺ (100)

### 4. 3-Allyl-4-methyl-2(3H)-thiazolon-1-(methylethyliden)-hydrazon

Aus 4-Allyl-3-thiosemicarbazid und Chloraceton
Ausbeute: 0,32 g (39 % der Theorie)
ESI-MS: 210 [M+H]⁺ (100)

### 5. 3-Allyl-4-phenyl-2(3H)-thiazolon-1-(methylethyliden)-hydrazon

Aus 4-Allyl-3-thiosemicarbazid und Phenacylchlorid
Ausbeute: 0,57 g (53 % der Theorie)
ESI-MS: 272 [M+H]⁺ (100)

### 6. 3-Allyl-4-tert-butyl-2(3H)-thiazolon-1-(methylethyliden)-hydrazon

Aus 4-Allyl-3-thiosemicarbazid und 1-Chlor-3,3-dimethyl-2-butanon
Ausbeute: 0,53 g (53 % der Theorie)
ESI-MS: 252 [M+H]⁺ (100)

### 7. 3,4-Diphenyl-2(3H)-thiazolon-1-(methylethyliden)-hydrazon

Aus 4-Phenyl-3-thiosemicarbazid und Phenacylchlorid
Ausbeute: 0,83 g (68 % der Theorie)
ESI-MS: 308 [M+H]⁺ (100)

### 8. {2-[(1-methylethyliden)-hydrazono]-3-phenyl-2,3-dihydro-1,3-thiazol-4-yl}essigsäure ethylester

Aus 4-Phenyl-3-thiosemicarbazid und 4-Chlor-acetessigsäure-ethylester
Ausbeute: 0,34 g (27 % der Theorie)
ESI-MS: 318 [M+H]⁺ (100)

### 9. 3,4,5-Trimethyl-2(3H)-thiazolon-1-(methylethyliden)-hydrazon

Aus 4-Methyl-3-thiosemicarbazid und 3-Chlor-2-butanon
Ausbeute: 0,27 g (34 % der Theorie)
ESI-MS: 198 [M+H]⁺ (100)

### Beispiele 10-15: Färbemittel mit 3,4-Dimethyl-2(3H)-thiazolon-1-(methylethyliden)-hydrazon

### Komponente (A1)

| | |
|---|---|
| 4,00 g | Decylpolyglucose (Plantaren® 2000), 50 %ige wässrige Lösung |
| 0,20 g | Ethylendiaminotetraessigsäure-Dinatriumsalz-Hydrat |
| 5,00 g | Ethanol |
| 0,46 g | 3,4-Dimethyl-2(3H)-thiazolon-1-(methylethyliden)-hydrazon |
| ad 100,00 g | Wasser, entmineralisiert |

### Komponente (A2)

| | |
|---|---|
| Y g | Kupplersubstanz gemäß Tabelle 1 |
| 0,80 g | Kaliumpersulfat |

Bei Raumtemperatur (20-25 °C) oder unter leichtem Erwärmen (35-40 °C) werden die vorstehend genannten Komponenten (A1) und (A2) homogen miteinander vermischt. Der pH-Wert des so erhaltenen gebrauchsfertigen Färbemittels (A) wird -falls erforderlich- mit Natronlauge, Natriumcarbonat, Ammoniak auf den in der Tabelle 1 angegebenen Wert eingestellt.

Das gebrauchsfertige Haarfärbemittel wird auf gebleichtes Büffelhaar aufge-tragen und mit einem Pinsel gleichmäßig verteilt. Nach einer Einwirkungszeit von 30 Minuten bei 40 °C wird das Haar mit einem handelsüblichen Shampoo gewaschen und sodann getrocknet.

Die Einsatzmenge der Kupplersubstanz sowie die erhaltenen Färbungen sind in der nachfolgenden Tabelle 1 zusammengefaßt.

**Tabelle 1:**

| **Bsp. Nr.** | **Verwendete Kupplersubstanz (Menge in g)** | **pH-Wert** | **Farbton** |
|---|---|---|---|
| **10** | 1,3-Diamino-benzol (0,27 g) | 9,9 | bordeauxrot |
| **11** | 2,4-Diamino-1-(2-hydroxyethoxy)-benzol sulfat (0,67 g) | 10,0 | dunkel braun-violett |
| **12** | N-(3-Dimethylamino-phenyl)-harnstoff (0,44 g) | 9,5 | blau |
| **13** | 3-Aminophenol (0,27 g) | 9,6 | himberrot |
| **14** | 5-Amino-2-methyl-phenol (0,31 g) | 9,6 | rot-orange |
| **15** | 1,3-Dihydroxybenzol (0,27 g) | 9,0 | rot |

### Beispiele 16 - 55: Färbemittel mit 2(3H)-Thiazolon-1-(methylethyliden)-hydrazonen der Formel (1)

### Komponente (A1)

| | |
|---|---|
| 4,00 g | Decylpolyglucose (Plantaren® 2000), 50 %ige wässrige Lösung |
| 0,20 g | Ethylendiaminotetraessigsäure-Dinatriumsalz-Hydrat |
| 5,00 g | Ethanol |
| X g | 2(3H)-Thiazolon-1-(methylethyliden)-hydrazon der Formel (1) |
| ad 100,00 g | Wasser, entmineralisiert |

### Komponente (A2)

| | |
|---|---|
| Y g | Kupplersubstanz gemäß Tabelle 2 |
| 0,80 g | Kaliumpersulfat |

Bei Raumtemperatur (20-25 °C) oder unter leichtem Erwärmen (35-40 °C) werden die vorstehend genannten Komponenten (A1) und (A2) homogen miteinander vermischt. Der pH-Wert des gebrauchsfertigen Färbemittels (A) wird -falls erforderlich- mit Natronlauge, Natriumcarbonat, Ammoniak auf den in der Tabelle 2 angegebenen Wert eingestellt.

Das gebrauchsfertige Haarfärbemittel wird auf gebleichtes Haar aufgetragen und mit einem Pinsel gleichmäßig verteilt. Nach einer Einwirkungszeit von 30 Minuten bei 40 °C wird das Haar mit einem handelsüblichen Shampoo gewaschen und sodann getrocknet.

Die Einsatzmenge der 2(3H)-Thiazolon-1-(methylethyliden)-hydrazonen der Formel (I), und der Kupplersubstanz sowie die erhaltenen Färbungen sind in der nachfolgenden Tabelle 2 zusammengefaßt.

**Tabelle 2:**

| **Bsp. Nr.** | **Derivat der Formel (I) gemäss Beispiel Nr. (Menge in g)** | **Verwendete Kupplersubstanz (Menge in g)** | **pH-Wert** | **Farbton** |
|---|---|---|---|---|
| **16** | Beispiel 2 (0,61 g) | 1,3-Diamino-benzol (0,27 g) | 9,6 | bordeauxrot |
| **17** | Beispiel 3 (0,56 g) | 1,3-Diamino-benzol (0,27 g) | 9,6 | bordeauxrot |
| **18** | Beispiel 4 (0,52 g) | 1,3-Diamino-benzol (0,27 g) | 9,6 | bordeauxrot |
| **19** | Beispiel 5 (0,68 g) | 1,3-Diamino-benzol (0,27 g) | 9,6 | leicht bordeauxrot |
| **20** | Beispiel 6 (0,63 g) | 1,3-Diamino-benzol (0,27 g) | 9,6 | bordeauxrot |
| **21** | Beispiel 7 (0,77 g) | 1,3-Diamino-benzol (0,27 g) | 9,6 | grau beige |
| **22** | Beispiel 8 (0,79 g) | 1,3-Diamino-benzol (0,27 g) | 9,6 | leicht bordeauxrot |
| **23** | Beispiel 9 (0,49 g) | 1,3-Diamino-benzol (0,27 g) | 9,6 | bordeauxrot |
| **24** | Beispiel 2 (0,61 g) | 2,4-Diamino-1-(2-hydroxyethoxy)-benzol sulfat (0,67 g) | 9,6 | dunkel bordeauxrot |
| **25** | Beispiel 3 (0,56 g) | 2,4-Diamino-1-(2-hydroxyethoxy)-benzol sulfat (0,67 g) | 9,6 | dunkel bordeauxrot |
| **26** | Beispiel 4 (0,52 g) | 2,4-Diamino-1-(2-hydroxyethoxy)-benzol sulfat (0,67 g) | 9,6 | dunkel bordeauxrot |
| **27** | Beispiel 5 (0,68 g) | 2,4-Diamino-1-(2-hydroxyethoxy)-benzol sulfat (0,67 g) | 9,6 | dunkel bordeauxrot |
| **28** | Beispiel 6 (0,63 g) | 2,4-Diamino-1-(2-hydroxyethoxy)-benzol sulfat (0,67 g) | 9,6 | dunkel bordeauxrot |
| **29** | Beispiel 7 (0,77 g) | 2,4-Diamino-1-(2-hydroxyethoxy)-benzol sulfat (0,67 g) | 9,6 | braun |
| **30** | Beispiel 8 (0,79 g) | 2,4-Diamino-1-(2-hydroxyethoxy)-benzol sulfat (0,67 g) | 9,6 | dunkel bordeauxrot |
| **31** | Beispiel 9 (0,49 g) | 2,4-Diamino-1-(2-hydroxyethoxy)-benzol sulfat (0,67 g) | 9,6 | dunkel bordeauxrot |
| **32** | Beispiel 2 (0,61 g) | N-(3-Dimethylamino-phenyl)-harnstoff (0,44 g) | 9,5 | leicht blau |
| **33** | Beispiel 3 (0,56 g) | N-(3-Dimethylamino-phenyl)-harnstoff (0,44 g) | 9,5 | leicht blau |
| **34** | Beispiel 4 (0,52 g) | N-(3-Dimethylamino-phenyl)-harnstoff (0,44 g) | 9,5 | blau |
| **35** | Beispiel 5 (0,68 g) | N-(3-Dimethylamino-phenyl)-harnstoff (0,44 g) | 9,5 | leicht blau |
| **36** | Beispiel 6 (0,63 g) | N-(3-Dimethylamino-phenyl)-harnstoff (0,44 g) | 9,5 | leicht blau |
| **37** | Beispiel 7 (0,77 g) | N-(3-Dimethylamino-phenyl)-harnstoff (0,44 g) | 9,5 | sehr leicht blau |
| **38** | Beispiel 8 (0,79 g) | N-(3-Dimethylamino-phenyl)-harnstoff (0,44 g) | 9,5 | leicht blau |
| **39** | Beispiel 9 (0,49 g) | N-(3-Dimethylamino-phenyl)-harnstoff (0,44 g) | 9,5 | leicht blau |
| **40** | Beispiel 2 (0,61 g) | 3-Aminophenol (0,27 g) | 9,6 | dunkel-blond |
| **41** | Beispiel 3 (0,56 g) | 3-Aminophenol (0,27 g) | 9,6 | himberrot |
| **42** | Beispiel 4 (0,52 g) | 3-Aminophenol (0,27 g) | 9,6 | himberrot |
| **43** | Beispiel5 (0,68 g) | 3-Aminophenol (0,27 g) | 9,6 | kupfer-braun |
| **44** | Beispiel 6 (0,63 g) | 3-Aminophenol (0,27 g) | 9,6 | himberrot |
| **45** | Beispiel 7 (0,77 g) | 3-Aminophenol (0,27 g) | 9,6 | braun |
| **46** | Beispiel 8 (0,79 g) | 3-Aminophenol (0,27 g) | 9,6 | leicht himberrot |
| **47** | Beispiel 9 (0,49 g) | 3-Aminophenol (0,27 g) | 9,6 | himberrot |
| **48** | Beispiel 2 (0,61 g) | 1,3-Dihydroxybenzol (0,27 g) | 10,0 | leicht rot-orange |
| **49** | Beispiel 3 (0,56 g) | 1,3-Dihydroxybenzol (0,27 g) | 10,0 | leicht rot-orange |
| **50** | Beispiel 4 (0,52 g) | 1,3-Dihydroxybenzol (0,27 g) | 10,0 | rot orange |
| **51** | Beispiel 5 (0,68 g) | 1,3-Dihydroxybenzol (0,27 g) | 10,0 | sehr leicht rot-orange |
| **52** | Beispiel 6 (0,63 g) | 1,3-Dihydroxybenzol (0,27 g) | 10,0 | rot-orange |
| **53** | Beispiel 7 (0,77 g) | 1,3-Dihydroxybenzol (0,27 g) | 10,0 | beige |
| **54** | Beispiel 8 (0,79 g) | 1,3-Dihydroxybenzol (0,27 g) | 10,0 | leicht rot-orange |
| **55** | Beispiel 9 (0,49 g) | 1,3-Dihydroxybenzol (0,27 g) | 10,0 | Rot-orange |

Alle in der vorliegenden Anmeldung angegebenen Prozentzahlen stellen -soweit nicht anders angegeben- Gewichtsprozente dar.

## Patentansprüche

1. Mittel zur Färbung von Fasern (A), **dadurch gekennzeichnet, dass** es (a) mindestens ein Hydrazon-Derivat der Formel (I) oder dessen physiologisch verträgliches Salz, 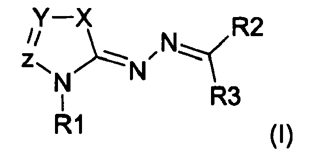 worin
**X** gleich Sauerstoff, Schwefel oder N-R4 ist;
**Y** gleich C-R5 oder Stickstoff ist;
**Z** gleich C-R6 oder Stickstoff ist;
mit der Bedingung, dass der heterozyklische Teil der Verbindung der Formel (I) maximal drei Heteroatome enthält;
**R1** und **R4** gleich oder verschieden sein können, und unabhängig voneinander eine gesättigte oder ungesättigte (C₁-C₁₂)-Alkylgruppe, eine mit einem Halogenatom substituierte (C₁-C₁₂)-Alkylgruppe, eine Hydroxy-(C₁-C₁₂)-alkylgruppe, eine Amino-(C₁-C₁₂)-alkylgruppe, eine Sulfonsäure-(C₁-C₁₂)-alkylgruppe, eine Formylgruppe, eine C(O)-(C₁-C₁₂)-Alkyl-gruppe, eine C(O)-Phenylgruppe, eine C(O)NH-)-(C₁-C₁₂)-Alkylgruppe, eine C(O)NH-Phenyl-gruppe, eine substituierte oder unsubstituierte Phenylgruppe oder eine Benzylgruppe darstellen;
**R2** und **R3** gleich oder verschieden sein können und unabhängig voneinander eine gesättigte oder ungesättigte (C₁-C₁₂)-Alkylgruppe darstellen;
**R5** und **R6** gleich oder verschieden sein können und unabhängig voneinander Wasserstoff, ein Halogenatom, eine gesättigte oder ungesättigte (C₁-C₁₂)-Alkylgruppe, eine mit einem Halogenatom substituierte (C₁-C₁₂)-Alkylgruppe, eine (C₁-C₁₂)-Hydroxyalkyl-gruppe, eine (C₁-C₁₂)-Alkoxygruppe, eine Cyano-gruppe, eine Nitrogruppe, eine Aminogruppe, eine (C₁-C₁₂)-Alkylaminogruppe, eine (C₁-C₁₂)-Dialkyl-aminogruppe, eine Carbonsäure, eine C(O)O-(C₁-C₁₂)-Alkylgruppe, eine substituierte oder unsubstituierte C(O)O-Phenylgruppe, eine substituierte oder unsubstituierte Phenylgruppe oder eine Naphtylgruppe darstellen;
und wenn **Y** und **Z** gleich C-R3 und C-R4 sind, **R5** und **R6** gemeinsam mit dem Restmolekül ein heterozyklisches oder carbozyklisches, gesättigtes oder ungesättigtes, substituiertes oder unsubstituiertes Ringsystem bilden; (b) mindestens eine Kupplersubstanz oder deren physiologisch verträgliches Salz,
sowie (c) als oxidatives Mittel ein Persulfatsalz enthält.

2. Mittel nach Anspruch 1, **dadurch gekennzeichnet, dass X** gleich Schwefel ist, **Y** und **Z** gleich C-R5 und C-R6 sind, und **R2** und **R3** eine Methylgruppe darstellen.

3. Mittel nach Anspruch 1 oder 2, **dadurch gekennzeichnet, dass** das Hydrazon-Derivat der Formel (I) ausgewählt ist aus 3-Methyl-2(3H)-thiazolon-(1-methylethyliden)hydrazon, 3,4-Dimethyl-2(3H)-thiazolon-(1-methylethyliden)hydrazon, 4-tert-Butyl-3-methyl-2(3H)-thiazolon-(1-methylethyliden)hydrazon, 3-Methyl-4-phenyl-2(3H)-thiazolon-(1-methylethyliden)hydrazon, 3-Methyl-4-(4-tolyl)-2(3H)-thiazolon-(1-methylethyliden)hydrazon, 4-(4-Methoxy)phenyl-3-methyl-2(3H)-thiazolon-(1-methylethyliden)hydrazon, 4-(3-Hydroxyphenyl)-3-methyl-2(3H)-thiazolon-(1-methylethyliden)hydrazon, 4-(4-Ethoxyphenyl)-3-methyl-2(3H)-thiazolon-(1-methylethyliden)hydrazon, 4-(4-Bromphenyl)-3-methyl-2(3H)-thiazolon-(1-methylethyliden)hydrazon, 4-(3-Bromphenyl)-3-methyl-2(3H)-thiazolon-(1-methylethyliden)hydrazon, 4-(4-Chlorphenyl)-3-methyl-2(3H)-thiazolon-(1-methylethyliden)hydrazon, 4-(3-Chlorphenyl)-3-methyl-2(3H)-thiazolon-(1-methylethyliden)hydrazon, 3-Methyl-4-(4-nitrophenyl)-2(3H)-thiazolon-(1-methylethyliden)hydrazon, 3-Methyl-4-(3-nitrophenyl)-2(3H)-thiazolon-(1-methylethyliden)hydrazon, 4-([1,1'-Biphenyl]-4-yl)-3-methyl-2(3H)-thiazolon-(1-methylethyliden)-hydrazon, 2-[(1-Methylethyliden)hydrazono]-2,3-dihydro-3-methyl-4-thiazol-carbonsäureethylester, 3,4,5-Trimethyl-2(3H)-thiazolon-(1-methylethyliden)hydrazon, 3,4-Dimethyl-5-phenyl-2(3H)-thiazolon-(1-methylethyliden)hydrazon, 3,5-Dimethyl-4-phenyl-2(3H)-thiazolon-(1-methylethyliden)hydrazon, 3-Methyl-4,5-diphenyl-2(3H)-thiazolon-(1-methylethyliden)hydrazon, 5-Ethyl-3-methyl-4-phenyl-2(3H)-thiazolon-(1-methylethyliden)hydrazon, 4-(4-Bromphenyl)-3-methyl-5-phenyl-2(3H)-thiazolon-(1-methylethyliden)-hydrazon, 3-Methyl-5-phenyl-4-(4-tolyl)-2(3Hrthiazolon-(1-methylethyliden)hydrazon, 5-(4-Chlorphenyl)-4-phenyl-3-methyl-2(3H)-thiazolon-(1-methylethyliden)-hydrazon, 5-(4-Chlorphenyl)-4-(4-methoxyphenyl)-3-methyl-2(3H)-thiazolon-(1-methylethyliden)-hydrazon, 2-[(1-Methylethyliden)hydrazono]-2,3-dihydro-3,4-dimethyl-4-thiazol-carbonsäureethylester, 4-Amino-2-[(1-methylethyliden)hydrazono]-2,3-dihydro-3-methyl-5-thiazol-carbonitril, 3-Ethyl-4,5-dimethyl-2(3H)-thiazolon-(1-methylethyliden)hydrazon, 2-[(1-Methylethyliden)hydrazono]-2,3-dihydro-3-ethyl-4-methyl-thiazol-carbonsäureethylester, 5-Methyl-3-(1-methylethyl)-4-phenyl-2(3H)-thiazolon-(1-methylethyliden)-hydrazon, 4,5-Dimethyl-3-(1-methylethyl)- 2(3H)-thiazolon-(1-methylethyliden)-hydrazon, 3-(1-Methylethyl)-4,5-diphenyl-2(3H)-thiazolon-(1-methylethyliden)-hydrazon, 4-Methyl-3-propyl-2(3H)-thiazolon-(1-methylethyliden)-hydrazon, 4,5-Dimethyl-3-propyl-2(3H)-thiazolon-(1-methylethyliden)-hydrazon, 4,5-Diphenyl-3-propyl-2(3H)-thiazolon-(1-methylethyliden)-hydrazon, 3-Butyl-4,5-dimethyl-2(3H)-thiazolon-(1-methylethyliden)-hydrazon, 3-Butyl-4,5-diphenyl-2(3H)-thiazolon-(1-methylethyliden)-hydrazon, 4,5-Dimethyl-3-(2-methylpropyl)-2(3H)-thiazolon-(1-methylethyliden)-hydrazon, 3-(2-Methylpropyl)-4,5-diphenyl-2(3H)-thiazolon-(1-methylethyliden)-hydrazon, 3-Allyl-2(3H)-thiazolon-(1-methylethyliden)-hydrazon, 3-Allyl-4-methyl-2(3H)-thiazolon-(1-methylethyliden)hydrazon,3-Allyl-4-tert-butyl-2(3H)-thiazolon-(1-methylethyliden)hydrazon, 3-Allyl-4-phenyl-2(3H)-thiazolon-(1-methylethyliden)hydrazon, 3-Allyl-4,5-dimethyl-2(3H)-thiazolon-(1-methylethyliden)hydrazon, 3-Allyl-4,5-diphenyl-2(3H)-thiazolon-(1-methylethytiden)hydrazon, 3-Hydroxyethyl-2(3H)-thiazolon-(1-methylethyliden)hydrazon, 3-Hydroxyethyl-4-methyl-2(3H)-thiazolon-(1-methylethyliden)hydrazon, 3-Hydroxyethyl-4,5-dimethyl-2(3H)-thiazolon-(1-methylethyliden)hydrazon, 3-Aminoethyl-2(3H)-thiazolon-(1-methylethyliden)hydrazon, 3-Aminoethyl-4-methyl-2(3H)-thiazolon-(1-methylethyliden)hydrazon, 3-Aminoethyl-4,5-dimethyl-2(3H)-thiazolon-(1-methylethyliden)hydrazon, 3-Phenyl-2(3H)-thiazolon-(1-methylethyliden)-hydrazon, 3,4-Diphenyl-2(3H)-thiazolon-(1-methylethyliden)hydrazon, 4-Methyl-3-phenyl-2(3H)-thiazolon-(1-methylethyliden)hydrazon, 4-p-Biphenylyl-3-phenyl-2(3H)-thiazolon-(1-methylethyliden)hydrazon, 4-(4-Methoxy)phenyl-3-phenyl-2(3H)-thiazolon-(1-methylethyliden)-hydrazon, 4-tert-Butyl-3-phenyl-2(3H)-thiazolon-(1-methylethyliden)-hydrazon, 4,5-Dimethyl-3-phenyl-2(3H)-thiazolon-(1-methylethyliden)-hydrazon, 5-Methyl-3,4-diphenyl-2(3H)-thiazolon-(1-methylethyliden)-hydrazon, 3,4,5-Triphenyl-2(3H)-thiazolon-(1-methylethyliden)hydrazon, 4,5-Dimethyl-3-(phenylmethyl)-2(3H)-thiazolon-(1-methylethyliden)hydrazon, 4-Phenyl-3-(phenylmethyl)-2(3H)-thiazolon-(1-methylethyliden)hydrazon, 5-Methyl-4-phenyl-3-(phenylmethyl)-2(3H)-thiazolon-(1-methylethyliden)-hydrazon, 3-Methyl-4,5,6,7-tetrahydro-2(3H)-benzothiazolon-(1-methylethyliden)-hydrazon, 3-Methyl-2(3H)-benzothiazolon-(1-methylethyliden)hydrazon, 3,6-Dimethyl-2(3H)-benzothiazolon-(1-methylethyliden)hydrazon, 6-Chlor-3-methyl-2(3H)-benzothiazolon-(1-methylethyliden)hydrazon, 7-Chlor-3-methyl-2(3H)-benzothiazolon-(1-methylethyliden)hydrazon, 6-Hydroxy-3-methyl-2(3H)-benzothiazolon-(1-methylethyliden)hydrazon, 5-Methoxy-3-methyl-2(3H)-benzothiazolon-(1-methylethyliden)hydrazon, 7-Methoxy-3-methyl-2(3H)-benzothiazolon-(1-methylethyliden)hydrazon, 5,6-Dimethoxy-3-methyl-2(3H)-benzothiazolon-(1-methylethyliden)hydrazon, 5-Ethoxy-3-methyl-2(3H)-benzothiazolon-(1-methylethyliden)hydrazon, 6-Ethoxy-3-methyl-2(3H)-benzothiazolon-(1-methylethyliden)hydrazon, 3-Methyl-5-nitro-2(3H)-benzothiazolon-(1-methylethyliden)hydrazon, 3-Methyl-6-nitro-2(3H)-benzothiazolon-(1-methylethyliden)hydrazon, 5-Acetamido-3-methyl-2(3H)-benzothiazolon-(1-methylethyliden)hydrazon, 6-Acetamido-3-methyl-2(3H)-benzothiazolon-(1-methylethyliden)hydrazon, 5-Anilino-3-methyl-2(3H)-benzothiazolon-(1-methylethyliden)hydrazon, 6-Anilino-3-methyl-2(3H)-benzothiazolon-(1-methylethyliden)hydrazon, 2-[(1-Methylethyliden)hydrazono]-2,3-dihydro-3-methyl-6-benzothiazol-carbonsäure, 2-[(1-Methylethyliden)hydrazono]-2,3-dihydro-3-methyl-4-benzothiazol-sulfonsäure, 2-[(1-Methylethyliden)hydrazono]-2,3-dihydro-3-methyl-5-benzothiazol-sulfonsäure, 2-[(1-Methylethyliden)hydrazono]-2,3-dihydro-3-methyl-6-benzothiazol-sulfonsäure, 2-[(1-Methylethyliden)-hydrazono]-2,3-dihydro-3-methyl-7-benzothiazol-sulfonsäure, 2-[(1-Methylethyfiden)hydrazono]-2,3-dihydro-N,N-3-trimethyl-6-benzothiazol-sulfonsäureamid, [(2-[(1-Methylethyliden)hydrazono]-2,3-dihydro-3-methyl-6-benzothiazolyl)oxy]essigsäure-hydrazid, 3-Ethyl-2(3H)-benzothiazolon-(1-methylethyliden)hydrazon, 6-Ethoxy-3-ethyl-2(3H)-benzothiazolon-(1-methylethyliden)hydrazon, 3-Propyl-2(3H)-benzothiazolon-(1-methylidenyliden)hydrazon, 3-Butyl-2(3H)-benzothiazolon-(1-methylethyliden)hydrazon und 3-Hexyl-2(3H)-benzothiazolon-(1-methylethyliden)hydrazon.

4. Mittel nach einem der Ansprüche 1 bis 3, **dadurch gekennzeichnet, dass** die Kupplersubstanz ausgewählt ist aus N-(3-Dimethylaminophenyl)-harnstoff, 2,6-Diamino-pyridin, 2-Amino-4-[(2-hydroxyethyl)-amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxy-ethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methyl-amino)-pyridin, 2,6-Diamino-3,5-dimethoxypyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxyethyl)amino-toluol, 4-Hydroxyindol, 3-Dimethylaminophenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlorphenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)-amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzo-dioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

5. Mittel nach einem der Ansprüche 1 bis 4, **dadurch gekennzeichnet, dass** das Persulfatsalz ausgewählt ist aus Kaliumpersulfat, Natriumpersulfat und Ammoniumpersulfat.

6. Mittel nach einem der Ansprüche 1 bis 5, **dadurch gekennzeichnet, dass** es die Hydrazon-Derivate der Formel (I), sowie die Kupplersubstanzen und die Persulfatsalze jeweils in einer Gesamtmenge von 0,01 bis 10 Gewichtsprozent enthält.

7. Mittel nach einem der Ansprüche 1 bis 5, **dadurch gekennzeichnet, dass** es zusätzlich 0,01 bis 10 Gewichtsprozent eines physiologisch unbedenklichen, direktziehenden Farbstoffs aus der Gruppe der kationischen und anionischen Farbstoffe, der Dispersionsfarbstoffe, der Nitrofarbstoffe, der Azofarbstoffe, der Chinonfarbstoffe und der Triphenylmethanfarbstoffe enthält.

8. Mittel nach einem der Ansprüche 1 bis 7, **dadurch gekennzeichnet, dass** es einen pH-Wert von 6 bis 10 aufweist.

9. Mittel nach einem der Ansprüche 1 bis 8, **dadurch gekennzeichnet, dass** es ein Haarfärbemittel ist.

10. 2-Komponenten-Kit, bestehend aus einer Farbträgermasse (A1), welche die Verbindung der Formel (I) enthält, und einer weiteren Farbträgermasse (A2), welche die Kupplersubstanzen und die Persulfatsalze enthält, sowie gegebenenfalls einem Mittel zur Einstellung des pH-Wertes.

11. 2-Komponenten-Kit, dessen 1. Komponente aus einem die Verbindungen der Formel (I), die Kupplersubstanzen und die Persulfatsalze sowie gegebenenfalls weitere übliche pulverförmige kosmetische Zusatzstoffe enthaltenden Pulver besteht, sofern die Verbindungen der Formel (I), die Kupplersubstanzen und das Persulfatsalz in fester Form vorliegen, und dessen 2. Komponente Wasser oder eine flüssige kosmetische Zubereitung ist, sowie gegebenenfalls einem Mittel zur Einstellung des pH-Wertes enthält.

12. Verfahren zum Färben von Haaren bei dem ein Färbemittel nach einem der Ansprüche 1 bis 9 auf die Haare aufgetragen wird und nach einer Einwirkungszeit von 5 bis 60 Minuten bei einer Temperatur von 20 bis 50 °C das Haar mit Wasser gespült, gegebenenfalls mit einem Shampoo gewaschen und sodann getrocknet wird.

13. Verfahren zum Färben von Haaren bei dem das gebrauchsfertige Färbemittel (A) unmittelbar vor der Anwendung durch Vermischen zweier Komponenten (A1) und (A2) -gegebenenfalls unter Zusatz eines Alkalisierungsmittels oder einer Säure-, hergestellt und sodann auf die Haare aufgetragen wird und nach einer Einwirkungszeit von 5 bis 60 Minuten bei einer Temperatur von 20 bis 50 °C das Haar mit Wasser gespült, gegebenenfalls mit einem Shampoo gewaschen und sodann getrocknet wird, **dadurch gekennzeichnet, dass** ein durch Vermischen zweier Komponenten (A1) und (A2) erhältliches Färbemittel (A) gemäß Anspruch 10 verwendet wird.

## Claims

1. Agent for dyeing fibres (A), **characterized in that** it comprises (a) at least one hydrazone derivative of the formula (I) or physiologically compatible salt thereof, 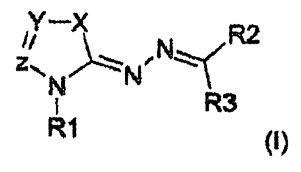 in which
**X** is oxygen, sulphur or N-R4;
**Y** is C-R5 or nitrogen;
**Z** is C-R6 or nitrogen;
with the proviso that the heterocyclic part of the compound of the formula (I) comprises at most three heteroatoms;
**R1** and **R4** may be identical or different and, independently of one another, are a saturated or unsaturated (C₁-C₁₂)-alkyl group, a (C₁-C₁₂)-alkyl group substituted by a halogen atom, a hydroxy-(C₁-C₁₂)-alkyl group, an amino (C₁-C₁₂)-alkyl group, a sulpho-(C₁-C₁₂)-alkyl group, a formyl group, a C(O)-(C₁-C₁₂)-alkyl group, a C(O)-phenyl group, a C(O)NH-)-(C₁-C₁₂)-alkyl group, a C(O)NH-phenyl group, a substituted or unsubstituted phenyl group or a benzyl group;
**R2** and **R3** may be identical or different and, independently of one another, are a saturated or unsaturated (C₁-C₁₂)-alkyl group;
**R5** and **R6** may be identical or different and, independently of one another, are hydrogen, a halogen atom, a saturated or unsaturated (C₁-C₁₂)-alkyl group, a (C₁-C₁₂)-alkyl group substituted by a halogen atom, a (C₁-C₁₂)-hydroxyalkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid, a C(O)O-(C₁-C₁₂)-alkyl group, a substituted or unsubstituted C(O)O-phenyl group, a substituted or unsubstituted phenyl group or a naphthyl group;
and when **Y** and **Z** are C-R3 and C-R4, **R5** and **R6**, together with the remainder of the molecule, form a heterocyclic or carbocyclic, saturated or unsaturated, substituted or unsubstituted ring system;
(b) at least one coupler substance or physiologically compatible salt thereof,
and (c) as oxidative agent, a persulphate salt.

2. Agent according to Claim 1, **characterized in that X** is sulphur, **Y** and **Z** are C-R5 and C-R6, and **R2** and **R3** are a methyl group.

3. Agent according to Claim 1 or 2, **characterized in that** the hydrazone derivative of the formula (I) is chosen from 3-methyl-2(3H)-thiazolone (1-methylethylidene)hydrazone, 3,4-dimethyl-2 (3H)-thiazolone (1-methylethylidene)hydrazone, 4-tert-butyl-3-methyl-2(3H)-thiazolone (1-methylethylidene)hydrazone, 3-methyl-4-phenyl-2(3H)-thiazolone (1-methylethylidene)hydrazone, 3-methyl-4-(4-tolyl)-2(3H)-thiazolone (1-methylethylidene)hydrazone, 4-(4-methoxy)phenyl-3-methyl-2(3H)-thiazolone (1-methylethylidene)hydrazone, 4-(3-hydroxyphenyl)-3-methyl-2(3H)-thiazolone (1-methylethylidene)hydrazone, 4-(4-ethoxyphenyl)-3-methyl-2(3H)-thiazolone (1-methylethylidene)hydrazone, 4-(4-bromophenyl)-3-methyl-2(3H)-thiazolone (1-methylethylidene)hydrazone, 4-(3-bromophenyl)-3-methyl-2(3H)-thiazolone (1-methylethylidene)-hydrazone, 4-(4-chlorophenyl)-3-methyl-2(3H)-thiazolone (1-methylethylidene)hydrazone, 4-(3-chlorophenyl)-3-methyl-2(3H)-thiazolone (1-methylethylidene)hydrazone, 3-methyl-4-(4-nitrophenyl)-2(3H)-thiazolone (1-methylethylidene)hydrazone, 3-methyl-4-(3-nitrophenyl)-2(3H)-thiazolone (1-methylethylidene)hydrazone, 4-([1,1'-biphenyl]-4-yl)-3-methyl-2(3H)-thiazolone (1-methylethylidene)hydrazone, 2-[(1-methylethylidene)-hydrazono]-2,3-dihydro-3-methyl-4-thiazolecarboxylic ethyl ester, 3,4,5-trimethyl-2(3H)-thiazolone (1-methylethylidene)hydrazone, 3,4-dimethyl-5-phenyl-2(3H)-thiazolone (1-methylethylidene)hydrazone, 3,5-dimethyl-4-phenyl-2(3H)-thiazolone (1-methylethylidene)hydrazone, 3-methyl-4,5-diphenyl-2(3H)-thiazolone (1-methylethylidene)hydrazone, 5-ethyl-3-methyl-4-phenyl-2 (3H)-thiazolone (1-methylethylidene)-hydrazone, 4-(4-bromophenyl)-3-methyl-5-phenyl-2(3H)-thiazolone (1-methylethylidene)hydrazone, 3-methyl-5-phenyl-4-(4-tolyl)-2(3H)-thiazolone (1-methylethylidene)hydrazone, 5-(4-chlorophenyl)-4-phenyl-3-methyl-2(3H)-thiazolone (1-methylethylidene)hydrazone, 5-(4-chlorophenyl)-4-(4-methoxyphenyl)-3-methyl-2(3H)-thiazolone (1-methylethylidene)hydrazone, 2-[(1-methylethylidene)hydrazono]-2,3-dihydro-3,4-dimethyl-4-thiazolecarboxylic ethyl ester, 4-amino-2-[(1-methylethylidene)hydrazono]-2,3-dihydro-3-methyl-5-thiazolecarbonitrile, 3-ethyl-4,5-dimethyl-2(3H)-thiazolone (1-methylethylidene)hydrazone, 2-[(1-methylethylidene)-hydrazono]-2,3-dihydro-3-ethyl-4-methylthiazolecarboxylic ethyl ester, 5-methyl-3-(1-methylethyl)-4-phenyl-2(3H)-thiazolone (1-methylethylidene)hydrazone, 4,5-dimethyl-3-(1-methylethyl)-2(3H)-thiazolone (1-methylethylidene)hydrazone, 3-(1-methylethyl)-4,5-diphenyl-2(3H)-thiazolone (1-methylethylidene)hydrazone, 4-methyl-3-propyl-2(3H)-thiazolone (1-methylethylidene)hydrazone, 4,5-dimethyl-3-propyl-2(3H)-thiazolone (1-methylethylidene)hydrazone, 4,5-diphenyl-3-propyl-2(3H)thiazolone (1-methylethylidene)hydrazone, 3-butyl-4,5-dimethyl-2(3H)-thiazolone (1-methylethylidene)hydrazone, 3-butyl-4,5-diphenyl-2(3H)-thiazolone (1-methylethylidene)hydrazone, 4,5-dimethyl-3-(2-methylpropyl)-2(3H)-thiazolone (1-methylethylidene)hydrazone, 3-(2-methylpropyl)-4,5-diphenyl-2(3H)-thiazolone (1-methylethylidene)hydrazone, 3-allyl-2(3H)-thiazolone (1-methylethylidene)hydrazone, 3-allyl-4-methyl-2(3H)-thiazolone (1-methylethylidene)-hydrazone, 3-allyl-4-tert-butyl-2(3H)-thiazolone (1-methylethylidene)hydrazone, 3-allyl-4-phenyl-2(3H)-thiazolone (1-methylethylidene)hydrazone, 3-allyl-4,5-dimethyl-2(3H)-thiazolone (1-methylethylidene)-hydrazone, 3-allyl-4,5-diphenyl-2(3H)-thiazolone (1-methylethylidene)hydrazone, 3-hydroxyethyl-2(3H)-thiazolone (1-methylethylidene)hydrazone, 3-hydroxyethyl-4-methyl-2(3H)-thiazolone (1-methylethylidene)-hydrazone, 3-hydroxyethyl-4,5-dimethyl-2(3H)-thiazolone (1-methylethylidene)hydrazone, 3-aminoethyl-2(3H)-thiazolone (1-methylethylidene)hydrazone, 3-aminoethyl-4-methyl-2(3H)-thiazolone (1-methylethylidene)-hydrazone, 3-aminoethyl-4,5-dimethyl-2(3H)-thiazolone (1-methylethylidene)hydrazone, 3-phenyl-2(3H)-thiazolone (1-methylethylidene)hydrazone, 3,4-diphenyl-2(3H)-thiazolone (1-methylethylidene)hydrazone, 4-methyl-3-phenyl-2(3H)-thiazolone (1-methylethylidene)hydrazone, 4-p-biphenylyl-3-phenyl-2(3H)-thiazolone (1-methylethylidene)hydrazone, 4-(4-methoxy)phenyl-3-phenyl-2(3H)-thiazolone (1-methylethylidene)hydrazone, 4-tert-butyl-3-phenyl-2(3H)-thiazolone (1-methylethylidene)hydrazone, 4,5-dimethyl-3-phenyl-2(3H)-thiazolone (1-methylethylidene)-hydrazone, 5-methyl-3,4-diphenyl-2(3H)-thiazolone (1-methylethylidene)hydrazone, 3,4,5-triphenyl-2(3H)-thiazolone (1-methylethylidene)hydrazone, 4,5-dimethyl-3-(phenylmethyl)-2(3H)-thiazolone (1-methylethylidene)-hydrazone, 4-phenyl-3-(phenylmethyl)-2(3H)-thiazolone (1-methylethylidene)hydrazone, 5-methyl-4-phenyl-3-(phenylmethyl)-2(3H)-thiazolone (1-methylethylidene)-hydrazone, 3-methyl-4,5,6,7-tetrahydro-2(3H)-benzothiazolone (1-methylethylidene)hydrazone, 3-methyl-2(3H)-benzothiazolone (1-methylethylidene)hydrazone, 3,6-dimethyl-2(3H)-benzothiazolone (1-methylethylidene)hydrazone, 6-chloro-3-methyl-2 (3H)-benzothiazolone (1-methylethylidene)hydrazone, 7-chloro-3-methyl-2 (3H)-benzothiazolone (1-methylethylidene)-hydrazone, 6-hydroxy-3-methyl-2(3H)-benzothiazolone (1-methylethylidene)hydrazone, 5-methoxy-3-methyl-2(3H)-benzothiazolone (1-methylethylidene)hydrazone, 7-methoxy-3-methyl-2(3H)-benzothiazolorie (1-methylethylidene)hydrazone, 5,6-dimethoxy-3-methyl-2(3H)-benzothiazolone (1-methylethylidene)hydrazone, 5-ethoxy-3-methyl-2(3H)-benzothiazolone (1-methylethylidene)hydrazone, 6-ethoxy-3-methyl-2(3H)-benzothiazolone (1-methylethylidene)hydrazone, 3-methyl-5-nitro-2(3H)-benzothiazolone (1-methylethylidene)-hydrazone, 3-methyl-6-nitro-2(3H)-benzothiazolone (1-methylethylidene)hydrazone, 5-acetamido-3-methyl-2(3H)-benzothiazolone (1-methylethylidene)hydrazone, 6-acetamido-3-methyl-2(3H)-benzothiazolone (1-methylethylidene)hydrazone, 5-anilino-3-methyl-2(3H)-benzothiazolone (1-methylethylidene)hydrazone, 6-anilino-3-methyl-2(3H)-benzothiazolone (1-methylethylidene)-hydrazone, 2-[(1-methylethylidene)hydrazono]-2,3-dihydro-3-methyl-6-benzothiazolecarboxylic acid, 2-[(1-methylethylidene)hydrazono]-2,3-dihydro-3-methyl-4-benzothiazolesulphonic acid, 2-[(1-methylethylidene)-hydrazono]-2,3-dihydro-3-methyl-5-benzothiazolesulphonic acid, 2-[(1-methylethylidene)hydrazono]-2,3-dihydro-3-methyl-6-benzothiazolesulphonic acid, 2-[(1-methylethylidene)hydrazono]-2,3-dihydro-3-methyl-7-benzothiazolesulphonic acid, 2-[(1-methylethylidene)-hydrazono]-2,3-dihydro-N,N-3-trimethyl-6-benzothiazolesulphonamide, [(2-[(1-methylethylidene)-hydrazono]-2,3-dihydro-3-methyl-6-benzothiazolyl)oxy]-acetic hydrazide, 3-ethyl-2(3H)-benzothiazolone (1-methylethylidene)hydrazone, 6-ethoxy-3-ethyl-2(3H)-benzothiazolone (1-methylethylidene)hydrazone, 3-propyl-2(3H)-benzothiazolone (1-methylethylidene)-hydrazone, 3-butyl-2(3H)-benzothiazolone (1-methylethylidene)hydrazone and 3-hexyl-2(3H)-benzothiazolone (1-methylethylidene)hydrazone.

4. Agent according to one of Claims 1 to 3, **characterized in that** the coupler substance is chosen from N-(3-dimethylaminophenyl)urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)-benzene, 1,3-diamino-4-(2,3-dihydroxypropoxy)benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3[di(2-hydroxyethyl)-amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]-aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]-phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

5. Agent according to one of Claims 1 to 4, **characterized in that** the persulphate salt is chosen from potassium persulphate, sodium persulphate and ammonium persulphate.

6. Agent according to one of Claims 1 to 5, **characterized in that** it comprises the hydrazone derivative of the formula (I), and the coupler substances and the persulphate salts, in each case in a total amount of from 0.01 to 10 per cent by weight.

7. Agent according to one of Claims 1 to 5, **characterized in that** it additionally comprises 0.01 to 10 per cent by weight of a physiologically acceptable, direct dye from the group of cationic and anionic dyes, disperse dyes, nitro dyes, azo dyes, quinone dyes and triphenylmethane dyes.

8. Agent according to one of Claims 1 to 7, **characterized in that** it has a pH of from 6 to 10.

9. Agent according to one of Claims 1 to 8, **characterized in that** it is a hair colourant.

10. Two-component kit, consisting of a colour carrier mass (A1) which comprises the compound of the formula (I), and a further colour carrier mass (A2) which comprises the coupler substances and the persulphate salts, and optionally an agent for adjusting the pH.

11. Two-component kit, whose first component consists of a powder comprising the compounds of the formula (I), the coupler substances and the persulphate salts, and optionally further customary pulverulent cosmetic additives, if the compounds of the formula (I), the coupler substances and the persulphate salt are in solid form, and whose second component is water or a liquid cosmetic preparation, and optionally comprises an agent for adjusting the pH.

12. Method for dyeing hair in which a colourant according to one of Claims 1 to 9 is applied to the hair and, after a contact time of from 5 to 60 minutes at a temperature of from 20 to 50°C, the hair is rinsed with water, optionally washed with a shampoo and then dried.

13. Method for dyeing hair in which the ready-to-use colourant (A) is prepared directly prior to use by mixing two components (A1) and (A2) - optionally with the addition of an alkalizing agent or an acid - and then applied to the hair and, after a contact time of from 5 to 60 minutes at a temperature of from 20 to 50°C, the hair is rinsed with water, optionally washed with a shampoo and then dried, **characterized in that** a colourant (A) according to Claim 10 obtainable by mixing two components (A1) and (A2) is used.

## Revendications

1. Composition (A) pour la teinture de fibres, **caractérisée en ce qu'**elle contient:
(a) au moins un dérivé d'hydrazine de formule (I) ou un sel physiologiquement acceptable d'un tel dérivé, 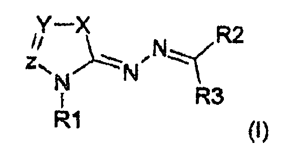 formule dans laquelle
**X** est un atome d'oxygène, de soufre ou N-R4 ;
**Y** est C-R5 ou un atome d'azote ;
**Z** est C-R6 ou un atome d'azote ;
à condition que la partie hétérocyclique du composé de formule (I) renferme au plus trois hétéroatomes ;
**R1** et **R4** peuvent être identiques ou différents et représentent, indépendamment l'un de l'autre, un groupe alkyle en C₁-C₁₂ saturé ou insaturé, un groupe alkyle en C₁-C₁₂ substitué par un atome d'halogène, un groupe hydroxyalkyle(C₁-C₁₂), un groupe aminoalkyle-(C₁-C₁₂), un groupe sulfoalkyle(C₁-C₁₂), un groupe formyle, un groupe C(O)-alkyle(C₁-C₁₂), un groupe C(O)-phényle, un groupe C(O)NH-)-alkyle(C₁-C₁₂), un groupe C(O)NH-phényle, un groupe phényle substitué ou non substitué ou un groupe benzyle ;
**R2** et **R3** peuvent être identiques ou différents et représentent, indépendamment l'un de l'autre, un groupe alkyle en C₁-C₁₂ saturé ou insaturé ;
**R5** et **R6** peuvent être identiques ou différents et représentent, indépendamment 1 'un de l'autre, un atome d'hydrogène, un atome d'halogène; un groupe alkyle en C₁-C₁₂ saturé ou insaturé, un groupe alkyle en C₁-C₁₂ substitué par un atome d'halogène, un groupe hydroxyalkyle en C₁-C₁₂, un groupe alcoxy en C₁-C₁₂, un groupe cyano, un groupe nitro, un groupe amino, un groupe alkyl(C₁-C₁₂)amino, un groupe dialkyl(C₁-C₁₂)-amino, un acide carboxylique, un groupe C(O)O-alkyle(C₁-C₁₂), un groupe C(O)O-phényle substitué ou non substitué, un groupe phényle substitué ou non substitué ou un groupe naphtyle ;
et quand **Y** et **Z** sont C-R3 et C-R4, **R5** et **R6** forment ensemble avec la molécule restante un système cyclique hétérocyclique ou carbocyclique, saturé ou insaturé, substitué ou non substitué;
(b) au moins un coupleur ou un sel physiologiquement acceptable de celui-ci ; ainsi que
(c) un persulfate en tant qu'agent oxydant.

2. Composition selon la revendication 1, **caractérisée en ce que X** est un atome de soufre, **Y** et **Z** sont C-R5 et C-R6, et R2 et R3 représentent un groupe méthyle.

3. Composition selon la revendication 1 ou 2, **caractérisée en ce que** le dérivé d'hydrazone de formule (I) est choisi parmi :
la 3-méthyl-2(3H)-thiazolone-(1-méthyléthylidène)-hydrazone,
la 3,4-diméthyl-2(3H)-thiazolone-(1-méthyléthylidène)hydrazone,
la 4-tert-butyl-3-méthyl-2(3H)-thiazolone-(1-méthyléthylidène)hydrazone,
la 3-méthyl-4-phényl-2(3H)-thiazolone-(1-méthyléthylidène)hydrazone,
la 3-méthyl-4-(4-tolyl)-2(3H)-thiazolone-(1-méthyléthylidène)hydrazone,
la 4-(4-méthoxy)phényl-3-méthyl-2(3H)-thiazolone-(1-méthyléthylidène)hydrazone,
la 4-(3-hydroxyphényl)-3-méthyl-2(3H)-thiazolone-(1-méthyléthylidène)hydrazone,
la 4-(4-éthoxyphényl)-3-méthyl-2(3H)-thiazolone-(1-méthyléthylidène)hydrazone,
la 4-(4-bromophényl)-3-méthyl-2(3H)-thiazolone-(1-méthyléthylidène)hydrazone,
la 4-(3-bromophényl)-3-méthyl-2(3H)-thiazolone-(1-méthyléthylidène)hydrazone,
la 4-(4-chlorophényl)-3-méthyl-2(3H)-thiazolone-(1-méthyléthylidène)hydrazone,
la 4-(3-chlorophényl)-3-méthyl-2(3H)-thiazolone-(1-méthyléthylidène)hydrazone,
la 3-méthyl-4-(4-nitrophényl)-2(3H)-thiazolone-(1-méthyléthylidène)hydrazone,
la 3-méthyl-4-(3-nitrophényl)-2(3H)-thiazolone-(1-méthyléthylidène)hydrazone,
la 4-([1,1'-biphényl]-4-yl)-3-méthyl-2(3H)-thiazolone-(1-méthyléthylidène)hydrazone,
le 2-[(1-méthyléthylidène)hydrazono]-2,3-dihydro-3-méthyl-4-thiazole-carboxylate d'éthyle,
la 3,4,5-triméthyl-2(3H)-thiazolone-(1-méthyléthylidène)hydrazone,
la 3,4-diméthyl-5-phényl-2(3H)-thiazolone-(1-méthyléthylidène)hydrazone,
la 3,5-diméthyl-4-phényl-2(3H)-thiazolone-(1-méthyléthylidène)hydrazone,
la 3-méthyl-4,5-diphényl-2(3H)-thiazolone-(1-méthyléthylidène)hydrazone,
la 5-éthyl-3-méthyl-4-phényl-2(3H)-thiazolone-(1-méthyléthylidène)hydrazone,
la 4-(4-bromophényl)-3-méthyl-5-phényl-2(3H)-thiazolone-(1-méthyléthylidène)hydrazone,
la 3-méthyl-5-phényl-4-(4-tolyl)-2(3H)-thiazolone-(1-méthyléthylidène)hydrazone,
la 5-(4-chlorophényl)-4-phényl-3-méthyl-2(3H)-thiazolone-(1-méthyléthylidène)hydrazone,
la 5-(4-chlorophényl)-4-(4-méthoxyphényl)-3-méthyl-2(3H)-thiazolone-(1-méthyléthylidène)-hydrazone,
le 2-[(1-méthyléthylidène)hydrazono]-2,3-dihydro-3,4-diméthyl-4-thiazole-carboxylate d'éthyle,
le 4-amino-2-[(1-méthyléthylidène)hydrazono]-2,3-dihydro-3-méthyl-5-thiazole-carbonitrile,
la 3-éthyl-4,5-diméthyl-2(3H)-thiazolone-(1-méthyléthylidène)hydrazone,
le 2-[(1-méthyléthylidène)hydrazono]-2,3-dihydro-3-éthyl-4-méthyl-thiazole-carboxylate d'éthyle,
la 5-méthyl-3-(1-méthyléthyl)-4-phényl-2(3H)-thiazolone-(1-méthyléthylidène)hydrazone,
la 4,5-diméthyl-3-(1-méthyléthyl)-2(3H)-thiazolone-(1-méthyléthylidène)hydrazone,
la 3-(1-méthyléthyl)-4,5-diphényl-2(3H)thiazolone-(1-méthyléthylidène)hydrazone,
la 4-méthyl-3-propyl-2(3H)-thiazolone-(1-méthyléthylidène)hydrazone,
la 4,5-diméthyl-3-propyl-2(3H)-thiazolone-(1-méthyléthylidène)hydrazone,
la 4,5-diphényl-3-propyl-2(3H)thiazolone-(1-méthyléthylidène)hydrazone,
la 3-butyl-4,5-diméthyl-2(3H)-thiazolone-(1-méthyléthylidène)hydrazone,
la 3-butyl-4,5-diphényl-2(3H)-thiazolone-(1-méthyléthylidène)hydrazone,
la 4,5-diméthyl-3-(2-méthylpropyl)-2(3H)-thiazolone-(1-méthyléthylidène)hydrazone,
la 3-(2-méthylpropyl)-4,5-diphényl-2(3H)-thiazolone-(1-méthyléthylidène)hydrazone,
la 3-allyl-2(3H)-thiazolone-(1-méthyléthylidène)-hydrazone,
la 3-allyl-4-méthyl-2(3H)-thiazolone-(1-méthyléthylidène)hydrazone,
la 3-allyl-4-tert-butyl-2(3H)-thiazolone-(1-méthyléthylidène)hydrazone,
la 3-allyl-4-phényl-2(3H)-thiazolone-(1-méthyléthylidène)hydrazone,
la 3-allyl-4,5-diméthyl-2(3H)-thiazolone-(1-méthyléthylidène)hydrazone,
la 3-allyl-4,5-diphényl-2(3H)-thiazolone-(1-méthyléthylidène)hydrazone,
la 3-hydroxyéthyl-2(3H)-thiazolone-(1-méthyléthylidène)hydrazone,
la 3-hydroxyéthyl-4-méthyl-2(3H)-thiazolone-(1-méthyléthylidène)hydrazone,
la 3-hydroxyéthyl-4,5-diméthyl-2(3H)-thiazolone-(1-méthyléthylidène)hydrazone,
la 3-aminoéthyl-2(3H)-thiazolone-(1-méthyléthylidène)hydrazone,
la 3-aminoéthyl-4-méthyl-2(3H)-thiazolone-(1-méthyléthylidène)hydrazone,
la 3-aminoéthyl-4,5-diméthyl-2(3H)-thiazolone-(1-méthyléthylidène)hydrazone,
la 3-phényl-2(3H)-thiazolone-(1-méthyléthylidène)-hydrazone,
la 3,4-diphényl-2(3H)-thiazolone-(1-méthyléthylidène)hydrazone,
la 4-méthyl-3-phényl-2(3H)-thiazolone-(1-méthyléthylidène)hydrazone,
la 4-p-biphénylyl-3-phényl-2(3H)-thiazolone-(1-méthyléthylidène)hydrazone,
la 4-(4-méthoxy)phényl-3-phényl-2(3H)-thiazolone-(1-méthyléthylidène)hydrazone,
la 4-tert-butyl-3-phényl-2(3H)-thiazolone-(1-méthyléthylidène)hydrazone,
la 4,5-diméthyl-3-phényl-2(3H)-thiazolone-(1-méthyléthylidène)hydrazone,
la 5-méthyl-3,4-diphényl-2(3H)-thiazolone-(1-méthyléthylidène)hydrazone,
la 3,4,5-triphényl-2(3H)-thiazolone-(1-méthyléthylidène)hydrazone,
la 4,5-diméthyl-3-(phénylméthyl)-2(3H)-thiazolone-(1-méthyléthylidène)hydrazone,
la 4-phényl-3-(phénylméthyl)-2(3H)-thiazolone-(1-méthyléthylidène)hydrazone,
la 5-méthyl-4-phényl-3-(phénylméthyl)-2(3H)-thiazolone-(1-méthyléthylidène)hydrazone,
la 3-méthyl-4,5,6,7-tétrahydro-2(3H)-benzothiazolone-(1-méthyléthylidène)hydrazone,
la 3-méthyl-2(3H)-benzothiazolone-(1-méthyléthylidène)hydrazone,
la 3,6-diméthyl-2(3H)-benzothiazolone-(1-méthyléthylidène)hydrazone,
la 6-chloro-3-méthyl-2(3H)-benzothiazolone-(1-méthyléthylidène)hydrazone,
la 7-chloro-3-méthyl-2(3H)-benzothiazolone-(1-méthyléthylidène)hydrazone,
la 6-hydroxy-3-méthyl-2(3H)-benzothiazolone-(1-méthyléthylidène)hydrazone,
la 5-méthoxy-3-méthyl-2(3H)-benzothiazolone-(1-méthyléthylidène)hydrazone,
la 7-méthoxy-3-méthyl-2(3H)-benzothiazolone-(1-méthyléthylidène)hydrazone,
la 5,6-diméthoxy-3-méthyl-2(3H)-benzothiazolone-(1-méthyléthylidène)hydrazone,
la 5-éthoxy-3-méthyl-2(3H)-benzothiazolone-(1-méthyléthylidène)hydrazone,
la 6-éthoxy-3-méthyl-2(3H)-benzothiazolone-(1-méthyléthylidène)hydrazone,
la 3-méthyl-5-nitro-2(3H)-benzothiazolone-(1-méthyléthylidène)hydrazone,
la 3-méthyl-6-nitro-2(3H)-benzothiazolone-(1-méthyléthylidène)hydrazone,
la 5-acétamido-3-méthyl-2(3H)-benzothiazolone-(1-méthyléthylidène)hydrazone,
la 6-acétamido-3-méthyl-2(3H)-benzothiazolone-(1-méthyléthylidène)hydrazone,
la 5-anilino-3-méthyl-2(3H)-benzothiazolone-(1-méthyléthylidène)hydrazone,
la 6-anilino-3-méthyl-2(3H)-benzothiazolone-(1-méthyléthylidène)hydrazone,
l'acide 2-[(1-méthyléthylidène)hydrazono]-2,3-dihydro-3-méthyl-6-benzothiazole-carboxylique,
l'acide 2-[(1-méthyléthylidène)hydrazono]-2,3-dihydro-3-méthyl-4-benzothiazole-sulfonique,
l'acide 2-[(1-méthyléthylidène)hydrazono]-2,3-dihydro-3-méthyl-5-benzothiazole-sulfonique,
l'acide 2-[(1-méthyléthylidène)hydrazono]-2,3-dihydro-3-méthyl-6-benzothiazole-sulfonique,
l'acide 2-[(1-méthyléthylidène)hydrazono]-2,3-dihydro-3-méthyl-7-benzothiazole-sulfonique,
le 2-[(1-méthyléthylidène)hydrazono]-2,3-dihydro-N,N-3-triméthyl-6-benzothiazole-sulfonamide,
le [(2-[(1-méthyléthylidène)hydrazono]-2,3-dihydro-3-méthyl-6-benzothiazolyl)oxy]-acétohydrazide,
la 3-éthyl-2(3H)-benzothiazolone-(1-méthyléthylidène)hydrazone,
la 6-éthoxy-3-éthyl-2(3H)-benzothiazolone-(1-méthyléthylidène)hydrazone,
la 3-propyl-2(3H)-benzothiazolone-(1-méthyléthylidène)hydrazone,
la 3-butyl-2(3H)-benzothiazolone-(1-méthyléthylidène)hydrazone, et
la 3-hexyl-2(3H)-benzothiazolone-(1-méthyléthylidène)hydrazone.

4. Composition selon l'une quelconque des revendications 1 à 3, **caractérisée en ce que** le coupleur est choisi parmi :
la N-(3-diméthylaminophényl)urée,
la 2,6-diaminopyridine,
le 2-amino-4-[(2-hydroxyéthyl)amino]anisole,
le 2,4-diamino-1-fluoro-5-méthylbenzène,
le 2,4-diamino-1-méthoxy-5-méthylbenzène,
le 2,4-diamino-1-éthoxy-5-méthylbenzène,
le 2,4-diamino-1-(2-hydroxyéthoxy)-5-méthylbenzène,
le 2,4-di[(2-hydroxyéthyl)amino]-1,5-diméthoxybenzène,
la 2,3-diamino-6-méthoxypyridine,
la 3-amino-6-méthoxy-2-(méthylamino)pyridine,
la 2,6-diamino-3,5-diméthoxypyridine,
la 3,5-diamino-2,6-diméthoxypyridine,
le 1,3-diaminobenzène,
le 2,4-diamino-1-(2-hydroxyéthoxy)benzène,
le 1,3-diamino-4-(2,3-dihydroxypropoxy)benzène,
le 1,3-diamino-4-(3-hydroxypropoxy)benzène,
le 1,3-diamino-4-(2-méthoxyéthoxy)benzène,
le 2,4-diamino-1,5-di(2-hydroxyéthoxy)benzène,
le 1-(2-aminoéthoxy)-2,4-diaminobenzène,
le 2-amino-1-(2-hydroxyéthoxy)-4-méthylaminobenzène,
l'acide 2,4-diaminophénoxyacétique,
la 3-[di(2-hydroxyéthyl)amino]aniline,
le 4-amino-2-di[(2-hydroxyéthyl)amino]-1-éthoxybenzène,
le 5-méthyl-2-(1-méthyléthyl)phénol,
la 3-[(2-hydroxyéthyl)amino]aniline,
la 3-[(2-aminoéthyl)amino]aniline,
le 1,3-di(2,4-diaminophénoxy)propane,
le di(2,4-diaminophénoxy)méthane,
le 1,3-diamino-2,4-diméthoxybenzène,
le 2,6-bis(2-hydroxyéthyl)aminotoluène,
le 4-hydroxyindole,
le 3-diméthylaminophénol,
le 3-diéthylaminophénol,
le 5-amino-2-méthylphénol,
le 5-amino-4-fluoro-2-méthylphénol,
le 5-amino-4-méthoxy-2-méthylphénol,
le 5-amino-4-éthoxy-2-méthylphénol,
le 3-amino-2,4-dichlorophénol,
le 5-amino-2,4-dichlorophénol,
le 3-amino-2-méthylphénol,
le 3-amino-2-chloro-6-méthylphénol,
le 3-aminophénol,
le 2-[(3-hydroxyphényl)amino]acétamide,
le 5-[(2-hydroxyéthyl)amino]-4-méthoxy-2-méthylphénol,
le 5-[(2-hydroxyéthyl)amino]-2-méthylphénol,
le 3-[(2-hydroxyéthyl)amino]phénol,
le 3-[(2-méthoxyéthyl)amino]phénol,
le 5-amino-2-éthylphénol,
le 5-amono-2-méthoxyphénol,
le 2-(4-amino-2-hydroxyphénoxy)éthanol,
le 5-[(3-hydroxypropyl)amino]-2-méthylphénol,
le 3-[(2,3-dihydroxypropyl)amino]-2-méthylphénol,
le 3-[(2-hydroxyéthyl)amino]-2-méthyl-phénol,
la 2-amino-3-hydroxypyridine,
la 2,6-dihydroxy-3,4-diméthylpyridine,
le 5-amino-4-chloro-2-méthylphénol,
le 1-naphtol,
le 2-méthyl-1-naphtol,
le 1,5-dihydroxynaphthalène,
le 1,7-dihydroxynaphthalène,
le 2,3-dihydroxynaphthalène,
le 2,7-dihydroxynaphthalène,
l'acétate de 2-méthyl-1-naphtol,
le 1,3-dihydroxybenzène,
le 1-chloro-2,4-dihydroxybenzène,
le 2-chloro-1,3-dihydroxybenzène,
le 1,2-dichloro-3,5-dihydroxy-4-méthylbenzène,
le 1,5-dichloro-2,4-dihydroxybenzène,
le 1,3-dihydroxy-2-méthylbenzène,
le 3,4-méthylènedioxyphénol,
la 3,4-méthylènedioxyaniline,
le 5-[(2-hydroxyéthyl)amino]-1,3-benzodioxole,
le 6-bromo-1-hydroxy-3,4-méthylènedioxybenzène,
l'acide 3,4-diaminobenzoïque,
la 3,4-dihydro-6-hydroxy-1,4(2H)-benzoxazine,
la 6-amino-3,4-dihydro-1,4(2H)-benzoxazine,
la 3-méthyl-1-phényl-5-pyrazolone,
le 5,6-dihydroxyindole,
la 5,6-dihydroxyindoline,
le 5-hydroxyindole,
le 6-hydroxyindole,
le 7-hydroxyindole, et
la 2,3-indolinedione.

5. Composition selon l'une quelconque des revendications 1 à 4, **caractérisée en ce que** le persulfate est choisi parmi le persulfate de potassium, le persulfate de sodium et le persulfate d'ammonium.

6. Composition selon l'une quelconque des revendications 1 à 5, **caractérisée en ce qu'**elle contient les dérivés d'hydrazone de formule (I) ainsi que les coupleurs et les persulfates chacun en une quantité totale de 0,01 à 10 % en poids.

7. Composition selon l'une quelconque des revendications 1 à 5, **caractérisée en ce qu'**elle contient en outre de 0,01 à 10 % en poids d'un colorant direct physiologiquement sans inconvénient, choisi dans le groupe des colorants cationiques et des colorants anioniques, des colorants en dispersion, des colorants nitrés, des colorants azoïques, des colorants quinoniques et des colorants triphénylméthane.

8. Composition selon l'une quelconque des revendications 1 à 7, **caractérisée en ce qu'**elle présente un pH de 6 à 10.

9. Composition selon l'une quelconque des revendications 1 à 8, **caractérisée en ce qu'**elle est une composition de teinture pour cheveux.

10. Nécessaire à 2 composants, constitué d'une matière colorante (A1) qui contient le composé de formule (I), et d'une autre matière colorante (A2) qui contient les coupleurs et les persulfates, ainsi qu'éventuellement d'un agent pour l'ajustement du pH.

11. Nécessaire à 2 composants, dont le premier composant consiste en une poudre contenant les composés de formule (I), les coupleurs et les persulfates ainsi qu'éventuellement d'autres adhésifs cosmétiques pulvérulents usuels, lorsque les composés de formule (I), les coupleurs et le persulfate se trouvent sous forme solide, et dont le deuxième composant est de l'eau ou une préparation cosmétique liquide, et contient éventuellement un agent pour l'ajustement du pH.

12. Procédé pour la teinture des cheveux, dans lequel on applique sur les cheveux une composition de teinture selon l'une quelconque des revendications 1 à 9 et, après un temps d'action de 5 à 60 minutes à une température de 20 à 50°C, on rince les cheveux à l'eau, éventuellement on les lave avec un shampooing et ensuite on les sèche.

13. Procédé pour la teinture des cheveux, dans lequel on prépare immédiatement avant l'emploi la composition de teinture (A) prête à l'emploi, par mélange de deux composants (A1) et (A2) - éventuellement avec addition d'un agent d'alcalinisation ou d'un acide - et ensuite on l'applique sur les cheveux et, après un temps d'action de 5 à 60 minutes à une température de 20 à 50°C, on rince les cheveux à l'eau, éventuellement on les lave avec un shampooing et ensuite on les sèche, **caractérisé en ce qu'**on utilise une composition de teinture (A) selon la revendication 10, pouvant être obtenue par mélange de deux composants (A1) et (A2).
